# EUROPEAN PATENT APPLICATION

(11) **EP 4 357 353 A1**
(43) Date of publication of application: **24.04.2024**
(21) Application number: 22825102.1
(22) Date of filing: 17.06.2022
(51) Int. Cl.: C07K 1/06, C07C 231/00, C07C 271/62

(54) **PEPTIDE COMPOUND PRODUCTION METHOD**

(30) Priority: 17.06.2021 JP 2021101016
(71) Applicant: Chubu University Educational Foundation, Kasugai-shi Aichi 487-8501 (JP)
(72) Inventor: YAMAMOTO, Hisashi, Kasugai-shi, Aichi 487-8501 (JP); MURAMATSU, Wataru, Kasugai-shi, Aichi 487-8501 (JP)
(74) Representative: Lavoix
(86) International application number: PCT/JP2022/024418
(87) International publication number: WO 2022/265115

(57) **Abstract**

A method of producing a polypeptide compound, wherein a peptide compound represented by formula (P) is obtained by inducing an amide formation reaction between an amino-protected lactam compound represented by formula (R1) and an amino acid ester or peptide ester compound represented by formula (R2).

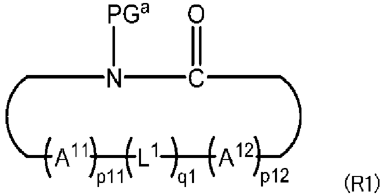

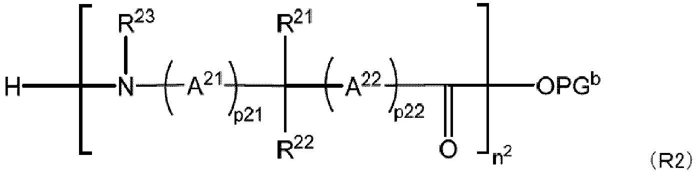

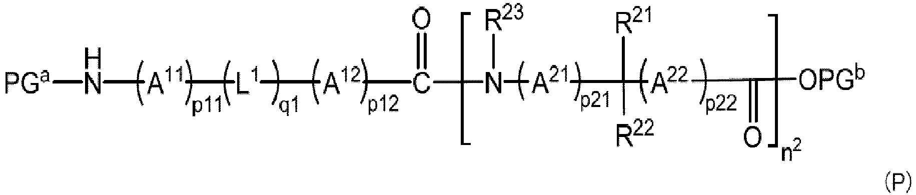

The definitions for the reference signs in formulae (R1), (R2), and (P) are as set forth in the claims.

## Description

### FIELD

The present invention relates to a novel method for producing a peptide compound.

### BACKGROUND

Conventionally, amide compounds represented by peptides have been used in a wide variety of fields, including pharmaceuticals, cosmetics, and functional foods. Development of synthetic methods thereof has been diligently pursued as an important research goal in synthetic chemistry (NPL 1 to 6). However, there are few truly effective catalysts or reactants other than carboxylic acid activators for the amidation reaction, which is the most important reaction in peptide synthesis. Therefore, it is unavoidable to use a reaction mode that forms by-products, and thus, peptide synthesis, which involves repeating multi-stage reactions, is extremely inefficient from the viewpoint of atom economy (atomic yield). The amount of by-products is large, and there are few effective purification means. As a result, the cost of disposal of by-products and purification constitutes most of the necessary costs for peptide synthesis, and is the largest obstacle to development in this field.

In peptide synthesis, which uses amino acids or derivatives thereof as starting materials, it is desirable for the amidation reaction to proceed with high stereoselectivity. Enzyme reactions in the body are examples of highly stereoselective amidation reactions. For example, in the body, peptides are synthesized with extremely high stereoselectivity through sophisticated use of enzymes and hydrogen bonds. However, enzyme reactions are not suitable for mass production, requiring enormous financial and time costs when applied to synthetic chemistry.

In synthetic chemistry, amidation reactions using catalysts have been examined, but in conventional means, the amide bond is formed primarily through the method of activating carboxylic acid, such that racemization occurs quickly, whereby synthesizing a peptide with high stereoselectivity and efficiency is difficult.

According to conventional methods, it is very difficult to link an additional amino acid or derivative to a peptide comprising a plurality of amino acids or derivatives thereof (chemical ligation) or link two or more peptides via amide bonds. As an amidation method for ligation to such peptides, there are known a method for ligation by using an amino acid having a sulfur atom to utilize the high reactivity of the sulfur atom (Non-Patent Literature 4) and a method for ligation by synthesizing an amino acid hydroxyamine to utilize the high reactivity of the hydroxyamine (Non-Patent Literature 5). However, in the former method, it is difficult to synthesize amino acids having a sulfur atom, and in the latter method, hydroxyamine synthesis involving several steps is necessary. Both methods are time-consuming and costly and have a disadvantage in efficiency.

The present inventors have developed, as techniques for synthesizing an amide compound in a highly chemoselective manner: a method of amidating a carboxylic acid/ester compound having a hydroxy group at the β-position in the presence of a specific metal catalyst (Patent Literature 1); a method of using a hydroxyamino/imino compound as an amino acid precursor and amidating it in the presence of a specific metal catalyst, and then reducing them in the presence of a specific metal catalyst (Patent Literature 2); and a method of amidating a carboxylic acid/ester compound in the presence of specific metal catalyst (Patent Literature 3). The present inventors have also developed: a method of synthesizing a peptide composed of various amino acid residues with high efficiency in a highly selective manner by causing an amide reaction between a carboxyl group of an N-terminal-protected amino acid/peptide and an amino group of a C-terminal-protected amino acid/peptide in the presence of specific silylation agent (optionally in combination with Lewis acid catalyst), followed by deprotection (Patent Literature 4); methods of synthesizing a peptide composed of various amino acid residues with high efficiency in a highly selective manner by causing an amide reaction between a carboxyl group of an N-terminal-protected or non-protected amino acid/peptide and an amino group of a C-terminal-protected or non-protected amino acid/peptide in the presence of a specific silylation agent, followed by deprotection (Patent Literatures 5 and 6); and a method of causing an amidation reaction using a Bronsted acid as a catalyst (Patent Literature 7). s

In recent years, attempts have been made to synthesize peptides using unprotected amino acids (Non-Patent Literatures 6 to 8). However, none of them have been satisfactory in terms of the types of amino acids available or reaction efficiency.

### CITATION LIST

### Patent Literature (PTL)

[Patent Literature 1] WO2017/204144 A
[Patent Literature 2] WO2018/199146 A
[Patent Literature 3] WO2018/199147 A
[Patent Literature 4] WO2019/208731 A
[Patent Literature 5] WO2021/085635 A
[Patent Literature 6] WO2021/085636 A
[Patent Literature 7] WO2021/149814 A

### Non-Patent Literature (NPL)

[Non-Patent Literature 1] Chem. Rev., 2011, Vol.111, p.6557-6602
[Non-Patent Literature 2] Org. Process Res. Dev., 2016, Vol.20, No.2, p.140-177
[Non-Patent Literature 3] Chem. Rev., 2016, Vol.116, p.12029-12122
[Non-Patent Literature 4] Science, 1992, Vol.256, p.221-225
[Non-Patent Literature 5] Angew. Chem. Int. Ed., 2006, Vol.45, p.1248-1252
[Non-Patent Literature 6] Chem. Eur. J. 2019, Vol.25, p.15091
[Non-Patent Literature 7] Org. Lett. 2020, Vol.22, p.8039
[Non-Patent Literature 8] Chem. Eur. J. 2018, Vol.24, p.7033
[Non-Patent Literature 9] Farren et al., J. Pept. Sci., 2009, 15:474-478

### SUMMARY

### Problem to be Solved

Against this background, there is a need for an inexpensive and efficient method for synthesizing peptide compounds composed of various amino acids. The present invention has been made in view of this objectives.

### Means for Solving the Problem

As a result of intensive investigations, the present inventors have found that peptide compounds composed of various amino acids can be synthesized inexpensively and efficiently by using an amino-protected lactam compound whose amino group is protected by a specific protective group along with an amino acid ester or peptide ester compound as substrates, and causing an amidation reaction between these compounds in a highly stereoselective manner and/or high efficiency, whereby the present inventors have arrived at the present invention.

Thus, the present invention provides the following aspects.

### [Aspect 1]

A method for producing a polypeptide compound represented by formula (P), comprising causing amide forming reaction between an amino-protected lactam compound represented by formula (R1) and an amino acid ester or peptide ester compound represented by formula (R2). 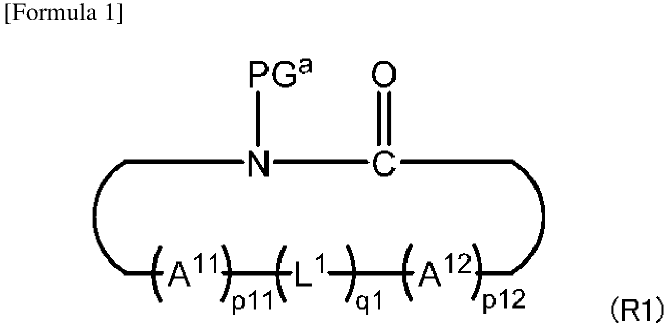 In formula (R1),
PG^{a} represents -C(=O)-O-R^{a}, -C(=O)-R^{a}, or -S(=O)₂-R^{a} a protective group represented by (where R^{a} represents a monovalent substituent.),
A¹¹and A¹² represents, independently of each other, a bivalent aliphatic hydrocarbon group having 1 to 5 carbon atoms which may optionally have one or more substituents,
p11 and p12 represents, independently of each other, 0 or 1,
L¹ represents a bivalent linking group,
q1 represents 0 or 1,
provided that the ring containing (A¹¹), (L¹), and (A¹²) is a 4- to 17-membered ring,
wherein the bivalent linking group L¹ may be combined with A¹¹ and/or A¹² to form a 4- to 8-membered heterocyclic ring that fuses with the lactam ring of formula (R1). 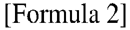 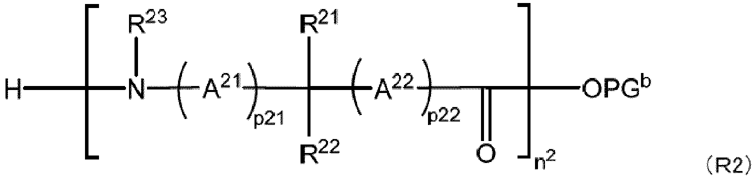 In formula (R2),
   PG^{b} represents a protective group for carboxyl groups,
   R²¹and R²² represents, independently of each other, a hydrogen atom, halogen atom, hydroxyl group, carboxyl group, nitro group, cyano group, or thiol group, and which may optionally have one or more substituents, amino group, monovalent aliphatic hydrocarbon group, monovalent aromatic hydrocarbon group, or monovalent heterocyclic group,
   R²³ represents a hydrogen atom, carboxyl group, hydroxyl group, and which may optionally have one or more substituents monovalent aliphatic hydrocarbon group, aromatic hydrocarbon group, or heterocyclic group, provided that when R²³ is a monovalent aliphatic hydrocarbon group, aromatic hydrocarbon group, or heterocyclic group, R²³ may be bound to the nitrogen atom via a linking group, or
   alternatively, R²¹ and R²³ may bind to each other and, along with the carbon atom to which R²¹ binds and the nitrogen atom to which R²³ binds, form a heterocyclic ring which may optionally have one or more substituents,
   A²¹ and A²² represents, independently of each other, a bivalent aliphatic hydrocarbon group having 1 to 3 carbon atoms which may optionally have one or more substituents,
   p21 and p22 represents, independently of each other, 0 or 1, and
   n² represents an integer of 1 or higher corresponding to the number of the structure units parenthesized with [ ], provided that when is n² equal to or greater than 2, then the two or more structure units in [ ] may be either identical to each other or different from each other. 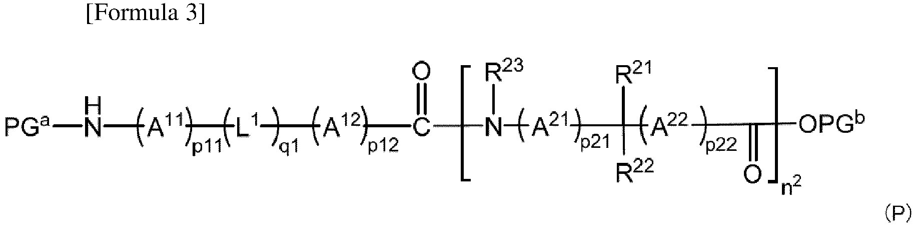 In formula (P),
      PG^{b}, A¹¹, A¹², L¹, p11, p12, and q1 each represent the same definition as that of the same symbol in formula (R1), and
      PG^{b}, R²¹, R²², R²³, A²¹, A²², p21, p22, and n² each represent the same definition as that of the same symbol in the formula (R2).

### [Aspect 2]

The method according to claim 1, wherein in formula (R1), PG^{a} is a protective group selected from a tert-butoxy carbonyl group (Boc), benzyloxycarbonyl group (Cbz), 2,2,2-trichloroethoxy carbonyl (Troc) group, allyoxycarbonyl (Alloc) group, 9-fluorenyl methyloxycarbonyl (Fmoc) group, 2-(trimethylsilyl)ethoxy carbonyl (Teoc) group, benzoyl (Bz) group, phthaloyl (Phth) group, paramethoxybenzoyl group (PMPCO), cinnamoyl group, toluene sulfonyl (Ts) group, ands 2- or 4-nitrobenzene sulfonyl (Ns) group.

### [Aspect 3]

The method according to claim 1 or 2, wherein in formula (R1), p11 is 1, p12 and q1 are both 0, A¹¹ represents a bivalent aliphatic hydrocarbon group having 2 to 6 carbon atoms which may optionally have one or more substituents.

### [Aspect 4]

The method according to claim 1 or 2, wherein in formula (R1), p11, p12, and q1 are all 1, A¹¹and A¹² are, independently of each other, a bivalent aliphatic hydrocarbon group having 1 to 3 carbon atoms which may optionally have one or more substituents, and L¹ is -C(=O)-N-.

### [Aspect 5]

The method according to any one of claims 1 to 4, wherein the molar ratio of the lactam compound of formula (R1) to the amino acid ester or peptide ester compound of formula (R2) used is from 1:5 to 5:1.

### [Aspect 6]

The method according to any one of claims 1 to 5, wherein the reaction is a batch reaction or a flow reaction.

### Effects of the Invention

The method according to the present invention makes it possible to synthesize peptide compounds composed of various amino acids inexpensively and efficiently by using an amino-protected lactam compound whose amino group is protected by a specific protective group along with an amino acid ester or peptide ester compound as substrates, and causing an amidation reaction between these compounds in a highly stereoselective manner and/or high efficiency.

### EMBODIMENTS

The present invention is described hereinafter in detail with reference to specific embodiments thereof. However, the present invention is not limited to the following embodiments and can be carried out in any embodiment that does not deviate from the gist of the present invention.

All the patent publication, unexamined patent publications, and non-patent literature cited in the present disclosure are incorporated herein by reference in their entireties for any purpose.

### [I. Definitions of terms]

The term "amino acid" herein refers to a compound having a carboxyl group and an amino group. Unless otherwise specified, the type of an amino acid is not particularly limited. For example, from the viewpoint of optical isomerism, an amino acid may be a D-amino acid, an L-amino acid, or a racemic amino acid. From the viewpoint of the relative positions of the carboxyl group and the amino group, an amino acid may be any of an α-amino acid, β-amino acid, γ-amino acid, δ-amino acid, or ε-amino acid. Examples of amino acids include, but are not limited to, natural amino acids that make up proteins. Examples include valine, leucine, isoleucine, alanine, arginine, glutamine, lysine, aspartic acid, glutamic acid, proline, cysteine, threonine, methionine, histidine, phenylalanine, tyrosine, tryptophan, asparagine, glycine, and serine.

The term "peptide" herein refers to a compound comprising a plurality of amino acids linked together via peptide bonds. Unless otherwise specified, the plurality of amino acid units constituting a peptide may be the same type of amino acid unit or may consist of two or more types of amino acid units. The number of amino acids constituting a peptide is not restricted as long as it is two or more. Examples include 2 (also called "dipeptide"), 3 (also called "tripeptide"), 4 (also called "tetrapeptide"), 5 (also called "pentapeptide"), 6, 7, 8, 9, 10, 15, 20, 30, 40, 50, 100, or more. Tripeptides and longer peptides may also be referred to by the term "polypeptide."

The term "amino group" herein refers to a functional group represented by any formula of -NH₂, -NRH, and -NRR' (where R and R' each represent a substituent) obtained by removing hydrogen from ammonia, a primary amine, and a secondary amine, respectively.

Unless otherwise specified, a hydrocarbon group herein may be either aliphatic or aromatic. An aliphatic hydrocarbon group may be in the form of either a chain or a ring. A chain hydrocarbon group may be linear or branched. A cyclic hydrocarbon group may be monocyclic, bridged cyclic, or spirocyclic. The hydrocarbon group may be saturated or unsaturated. In other words, one, two, or more carbon-carbon double and/or triple bonds may be included. Specifically, "hydrocarbon group" represents a concept including an alkyl group, alkenyl group, alkynyl group, cycloalkyl group, cycloalkenyl group, cycloalkynyl group, aryl group, etc. Unless otherwise specified, one, two, or more hydrogen atoms of the hydrocarbon group may be replaced with any substituents and one, two, or more carbon atoms of the hydrocarbon group may be replaced with any heteroatoms corresponding to the valence thereof.

The term "hydrocarbon oxy group" herein refers to a group comprising an oxy group (-O-) linked via one bond thereof to the hydrocarbon group as defined above. In other words, the hydrocarbon oxy groups herein encompass alkyloxy group, alkenyloxy group, alkynyloxy group, cycloalkyloxy group, cycloalkenyloxy group, cycloalkynyloxy group, and aryloxy group.

The term "hydrocarbon carbonyl group" herein refers to a group comprising a carbonyl group (-C(=O)-) linked via one bond thereof to the hydrocarbon group as defined above. In other words, hydrocarbon carbonyl groups herein encompass alkyl carbonyl group, alkenyl carbonyl group, alkynyl carbonyl group, cycloalkyl carbonyl group, cycloalkenyl carbonyl group, cycloalkynyl carbonyl group, and aryl carbonyl group.

The term "hydrocarbon sulfonyl group" herein refers to a group comprising a sulfonyl group (-S(=O)₂-) linked via one bond thereof to the hydrocarbon group as defined above. In other words, hydrocarbon sulfonyl groups herein encompass alkyl sulfonyl group, alkenyl sulfonyl group, alkynyl sulfonyl group, cycloalkyl sulfonyl group, cycloalkenyl sulfonyl group, cycloalkynyl sulfonyl group, and aryl sulfonyl group.

The term "heterocyclic group" herein may be saturated or unsaturated. In other words, it may contain one, two, or more carbon-carbon double and/or triple bonds. A heterocyclic group may be monocyclic, bridged cyclic, or spirocyclic. The heteroatom included in the constituent atoms of the heterocyclic group is not particularly limited, examples thereof including nitrogen, oxygen, sulfur, phosphorus, and silicon.

The term "heterocyclic ring oxy group" herein refers to a group comprising an oxy group (-O-) linked via one bond thereof to the heterocyclic group as defined above.

The term "heterocyclic ring carbonyl group" herein refers to a group comprising a carbonyl group (-C(=O)-) linked via one bond thereof to the heterocyclic group as defined above.

The term "heterocyclic ring sulfonyl group" herein refers to a group comprising a sulfonyl group (-S(=O)₂-) linked via one bond thereof to the heterocyclic group as defined above.

The term "metal-oxy group" (which may optionally have one or more substituents) herein refers to a group represented by formula (R)ₙ-M-O-, where M represents a metal element, R represents a substituent, and n represents an integer of from 0 to 8 which can be determined depending on the coordination number of the metal element M.

Unless otherwise specified, the term "substituent" herein refers, independently of each other, to any substituent which is not particularly limited so long as the amidation step of the production method according to the present invention proceeds. Examples include, but are not limited to, a halogen atom, hydroxyl group, carboxyl group, nitro group, cyano group, thiol group, sulfonic acid group, amino group, amide group, imino group, imide group, hydrocarbon group, heterocyclic group, hydrocarbon oxy group, hydrocarbon carbonyl group (acyl group), hydrocarbon oxycarbonyl group, hydrocarbon carbonyl oxy group, hydrocarbon substitution amino group, hydrocarbon substitution amino carbonyl group, hydrocarbon carbonyl substitution amino group, hydrocarbon substitution thiol group, hydrocarbon sulfonyl group, hydrocarbon oxysulfonyl group, hydrocarbon sulfonyl oxy group, heterocyclic oxy group, heterocyclic carbonyl group, heterocyclic oxycarbonyl group, heterocyclic carbonyl oxy group, heterocyclic amino group, heterocyclic amino carbonyl group, heterocyclic carbonyl substitution amino group, heterocyclic substitution thiol group, heterocyclic sulfonyl group, heterocyclic oxysulfonyl group, and heterocyclic sulfonyl oxy group. The term "substituents" also may include functional groups obtained by substituting any of the aforementioned functional groups with any of the aforementioned functional groups as long as the valence and physical properties thereof permit. When a functional group has one or more substituents, the number of the substituents is not particularly limited as long as the valence and physical properties thereof permit. When the functional group has two or more substituents, they may be identical to each other or different from each other.

Some of the abbreviations used herein are summarized in Tables 1-1 and 1-2 below.

**[Table 1-1]**

| Abbreviation | Definition |
|---|---|
| Ac | acetyl |
| acac | acetyl acetonate |
| Alloc | allyoxycarbonyl |
| Bn or Bzl | benzyl |
| Boc | tert-butoxy carbonyl |
| Bu | butyl |
| Bz | benzoyl |
| Cbz | benzyloxycarbonyl |
| Cp | cyclopentadienyl |
| 2,4-DNP | 2,4-dinitrophenyl |
| Et | ethyl |
| Fmoc | 9-fluorenyl methyloxycarbonyl |
| HOBt | 1-hydroxybenzotriazole |
| i-Pr | isopropyl |
| MABR | methyl aluminum bis(4-bromo-2,6-di-tert-butyl phenoxide) |
| Me | methyl |
| Ms | mesyl |
| Ns | 2- or 4-nitrobenzene sulfonyl |

**[Table 1-2]**

| Abbreviation | Definition |
|---|---|
| Pfp | pentafluorophenyl |
| Phth | phthaloyl |
| PMB | paramethoxybenzyl |
| PMPCO | paramethoxybenzoyl |
| Pr | propyl |
| TBAF | tetrabutyl ammonium fluoride |
| TBDPS | tert-butyl diphenylsilyl |
| TBS | tri-tert-butylsilyl |
| t-Bu | tert-butyl |
| TES | triethylsilyl |
| Tf | trifluoromethanesulfonyl |
| THF | tetrahydrofuran |
| TIPS | triisopropyl silyl |
| TMS | trimethylsilyl |
| TMS-IM | trimethylsilylimidazole |
| TMS-OTf | trifluoromethane sulfonic acid trimethylsilyl |
| Troc | 2,2,2-trichloroethoxy carbonyl |
| Trt | trityl |
| Ts | toluene sulfonyl |
| wscHCl | 1-ethyl-3-(3-dimethylaminopropyl)carbodiimide hydrochloride |

Amino acids and residues thereof may herein be represented by three-letter abbreviations well known to a person skilled in the art. The three-letter abbreviations of major amino acids are shown in the following table.

**[Table 2]**

| Abbreviation | Definition |
|---|---|
| Ala | alanine |
| Arg | arginine |
| Asn | asparagine |
| Asp | aspartic acid |
| Cys | cysteine |
| Gln | glutamine |
| Glu | glutamic acid |
| Gly | glycine |
| His | histidine |
| Ile | isoleucine |
| Leu | leucine |
| Lys | lysine |
| Met | methionine |
| Phe | phenylalanine |
| Phg | phenylglycine |
| Pro | proline |
| Ser | serine |
| Thr | threonine |
| Trp | tryptophan |
| Tyr | tyrosine |
| Val | valine |

β-homoamino acids and residues thereof may herein be represented by "Ho" followed by three-letter abbreviations of corresponding β-amino acids.

### [II. Production method of peptide compounds]

### * Summary

An aspect of the present invention relates to a method for producing a polypeptide compound represented by formula (P), comprising causing amide forming reaction between an amino-protected lactam compound represented by formula (R1) and an amino acid ester or peptide ester compound represented by formula (R2) (hereinafter also referred to as "the production method of a peptide compound according to the present invention" or simply as "the production method of the present invention") (the definitions of substituents in each formula will be explained below).

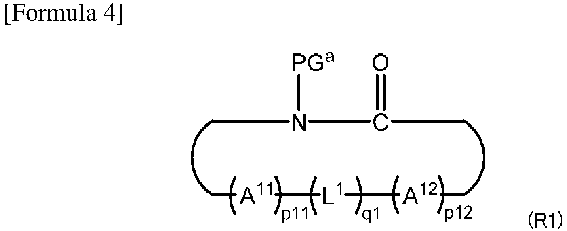

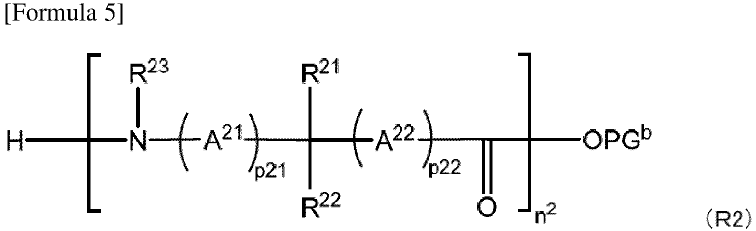

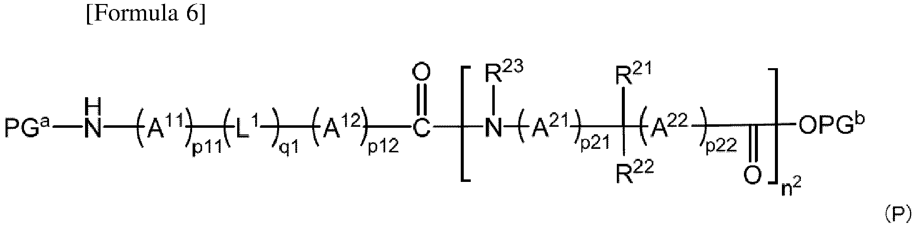

There have been examples in which an amino-protected lactam compounds such as N,N'-di-Boc-2,5-diketopiperazine is reacted with a nucleophilic reagent (an example is Non-Patent Literature 9: Farren et al., J. Pept. Sci., 2009, 15:474-478). However, there has been no example in which an amino-protected lactam compound is subjected to an amidation with an amino acid ester or peptide ester compound to synthesize a peptide ester compound. This is the first reaction discovered by the present inventors.

The amidation reaction in the production method of the present invention may have various advantages, including, but not limited to, the following.
* Due to the structural features of the substrate compound (R1) (lactam), racemisation through an oxazolone intermediate does not occur as observed in conventional peptide synthesis.
* If the substrate compound (R1) (lactam) and the substrate compound (R2) (amino acid ester or peptide ester) are used in 1:1 equivalents and the reaction proceeds in 100% yield, theoretically no by-products are produced (E-factor = 0, atom economy (atomic efficiency) = 100%).
* No reaction solvent is required (but a solvent may be used).
* No catalyst (e.g., Lewis acid catalyst) is required (but a catalyst may be used).

### *Substrate compound (R1)

The definition of each symbol in the above general formula (R1) is as follows.

PG^{a} represents a protective group for amino acids represented by -C(=O)-O-R^{a}, - C(=O)-R^{a}, or -S(=O)₂-R^{a}, where R^{a} represents a monovalent substituent. Preferable examples of R^{a} include monovalent hydrocarbon groups or heterocyclic groups which may optionally have one or more substituents. If these groups have substituents, the types of the possible substituents are as explained earlier. Examples of the number of substituents are, e.g., 5, 4, 3, 2, 1 or 0.

While protective groups for amino acids in general will be explained below, the protective group PG^{a} represented by -C(=O)-O-R^{a} corresponds to a non-substituted or substituted hydrocarbon or heterocyclic oxycarbonyl group (carbamate group). Specific examples thereof include, although are not limited to, tert-butoxy carbonyl (Boc) group, benzyloxycarbonyl (Cbzor Z) group, methoxy carbonyl group, ethoxy carbonyl group, 2-(trimethylsilyl)ethoxy carbonyl (Teoc) group, 2-phenyl ethoxy carbonyl group, 1-(1-adamantyl)-1-methyl ethoxy carbonyl group, 1-(3,5-di-t-butylphenyl)-1-methyl ethoxy carbonyl group, vinyloxycarbonyl group, allyoxycarbonyl (Alloc) group, N-hydroxy piperidinyl oxycarbonyl group, p-methoxybenzyloxycarbonyl group, p-nitrobenzyloxycarbonyl group, 2-(1,3-dithianyl)methoxy carbonyl, m-nitrophenoxycarbonyl group, 3,5-dimethoxybenzyloxycarbonyl group, o-nitrobenzyloxycarbonyl group, 2,2,2-trichloroethoxy carbonyl (Troc) group, and 9-fluorenyl methyloxycarbonyl (Fmoc) group.

The protective group PG^{a} represented by -C(=O)-R^{a} corresponds to a non-substituted or substituted hydrocarbon or heterocyclic acyl group. Specific examples thereof include, although are not limited to, benzoyl (Bz) group, orthomethoxybenzoyl group, 2,6-dimethoxybenzoyl group, paramethoxybenzoyl (PMPCO) group, phthaloyl (Phth) group, and cinnamoyl group.

The protective group PG^{a} represented by -S(=O)₂-R^{a} corresponds to a non-substituted or substituted hydrocarbon or heterocyclic sulfonyl group. Specific examples thereof include, although are not limited to, methanesulfonyl (Ms) group, toluene sulfonyl (Ts) group, and 2- or 4-nitrobenzene sulfonyl (Ns) group.

Preferable examples of the protective group PG^{a} include, although are not limited to, tert-butoxy carbonyl group (Boc), benzyloxycarbonyl group (Cbz), 2,2,2-trichloroethoxy carbonyl (Troc) group, allyoxycarbonyl (Alloc) group, 9-fluorenyl methyloxycarbonyl (Fmoc) group, 2-(trimethylsilyl)ethoxy carbonyl (Teoc) group, benzoyl (Bz) group, phthaloyl (Phth) group, paramethoxybenzoyl group (PMPCO), cinnamoyl group, toluene sulfonyl (Ts) group, and 2- or 4-nitrobenzene sulfonyl (Ns) group. These protecting groups are preferred since they can easily protect an amino group and can be removed under relatively mild conditions, as described above.

A¹¹and A¹² represent, independently of each other, a bivalent aliphatic hydrocarbon group having 1 to 5 carbon atoms which may optionally have one or more substituents. Specific examples include bivalent aliphatic hydrocarbon groups derived from monovalent aliphatic hydrocarbon groups cited for R²¹, R²², and R²³ of formula (R2) below, by removing any hydrogen atom therefrom. Other details are the same as those described below for R²¹, R²², and R²³ in formula (R2).

p11 and p12 represents, independently of each other, 0 or 1. In other words, p11 and p12 define the presence or absence of divalent aliphatic hydrocarbon groups A11 and A12, respectively, in the lactam ring of formula (R1). Specifically, when p11 is 0, the lactam ring of formula (R1) does not contain a bivalent aliphatic hydrocarbon group A¹¹, while when p11 is 1, the lactam ring of formula (R1) contains a bivalent aliphatic hydrocarbon group A¹¹. Likewise, when p12 is 0, the lactam ring of formula (R1) does not contain a bivalent aliphatic hydrocarbon group A¹², while when p12 is 1, the lactam ring of formula (R1) contains a bivalent aliphatic hydrocarbon group A¹².

L¹ represents a bivalent linking group. Such linking groups may be selected from, although are not limited to, independently of each other, the following structures. The direction of each linking group below is not limited to the direction shown in the chemical formula below. That is, the chemical bond shown on the right side of the chemical formula below may be linked to the (A¹¹) side of formula (R1) and the bonding hand shown on the left side may be linked to the (A¹²) side of formula (R1). Alternatively, the chemical bond shown on the right side of the chemical formula below may be linked to the (A¹²) side of formula (R1) and the bonding hand shown on the left side may be linked to the (A¹¹) side of formula (R1). In the formula below, R^{x} represents, independently of each other, a hydrogen atom, carboxyl group, or a monovalent hydrocarbon group or heterocyclic group which may optionally have one or more substituents. When any of the groups have one or more substituents, the types of substituents are as described previously. Examples of the number of substituents are, e.g., 5, 4, 3, 2, 1 or 0. Details of the monovalent hydrocarbon group or heterocyclic group are described below.

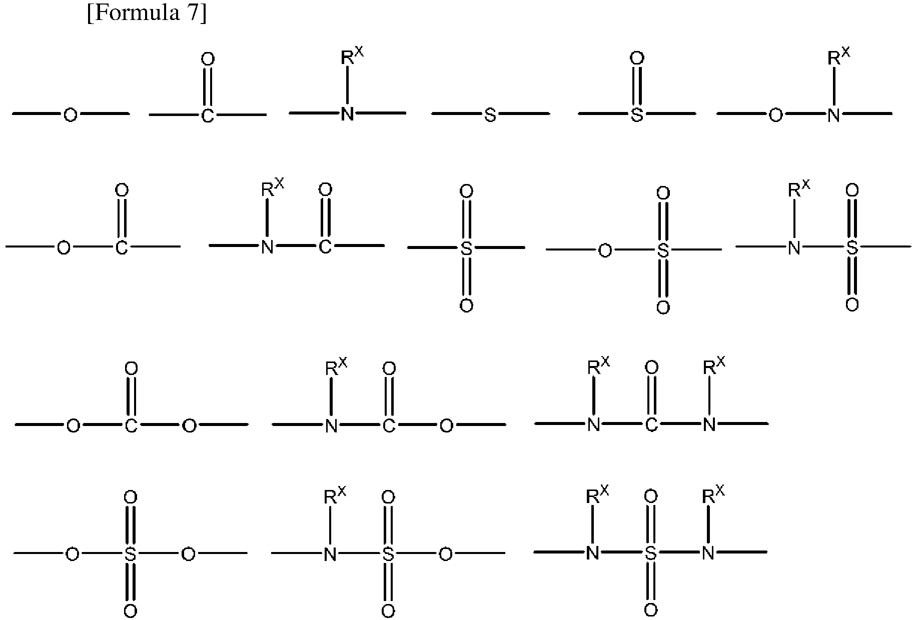

The bivalent linking group L¹ may be combined with A¹¹ and/or A¹² to form a 4-to 8-membered heterocyclic ring that fuses with the lactam ring of formula (R1). For example, when any of Example for the bivalent linking group L¹ mentioned above has, as R^{x}, a carboxyl group or a monovalent hydrocarbon group or heterocyclic group which may optionally have one or more substituents, this group may be combined with A¹¹ and/or A¹² to form a 4- to 8-membered heterocyclic ring that fuses with the lactam ring of formula (R1).

q1 represents 0 or 1. In other words, q1 defines the absence or presence of a bivalent linking group L¹ in the lactam ring of formula (R1). In other words, when q1 is 0, the lactam ring of formula (R1) does not contain a bivalent linking group L¹, while when q1 is 1, the lactam ring of formula (R1) contains a bivalent linking group L¹.

The number of members of the lactam ring containing (A¹¹), (L¹) and (A¹²) of formula (R1) may usually be from 4 to 17. In other words, according to an embodiment, the lactam ring of formula (R1) may be a 4-membered ring, 5-membered ring, 6-membered ring, 7-membered ring, 8-membered ring, 9-membered ring, 10-membered ring, 11-membered ring, 12-membered ring, 13-membered ring, 14-membered ring, 15-membered ring, 16-membered ring, or 17-membered ring. According to an embodiment, the lactam ring of formula (R1) may be a 4-membered ring, 5-membered ring, 6-membered ring, 7-membered ring, or 13-membered rings.

### *Substrate compound (R2)

The definition of each symbol in the above general formula (R2) is as follows.

R²¹ and R²² represent, independently of each other, a monovalent hydrocarbon group or heterocyclic group which may optionally have one or more substituents. If these groups have substituents, Example of such substituents are as described earlier. Examples of the number of substituents are, e.g., 5, 4, 3, 2, 1, or 0.

R²¹ and R²² represent, independently of each other, a hydrogen atom, halogen atom, hydroxyl group, carboxyl group, nitro group, cyano group, or thiol group, or a monovalent hydrocarbon group or heterocyclic group which may optionally have one or more substituents. If these groups have substituents, Example of such substituents are as described earlier. Examples of the number of substituents are, e.g., 5, 4, 3, 2, 1, or 0.

When each of R²¹ and/or R²² is a monovalent hydrocarbon group or heterocyclic group which may optionally have one or more substituents, a linking group may exist between the hydrocarbon group or heterocyclic group and the carbon atom to which it binds. Such linking groups may be selected from, although are not limited to, independently of each other, the following structures (in the formulae below, A represents, independently of each other, a monovalent hydrocarbon group or heterocyclic group which may optionally have one or more substituents. When two or more A's exist in the same groups, they may be identical to each other or different from each other.).

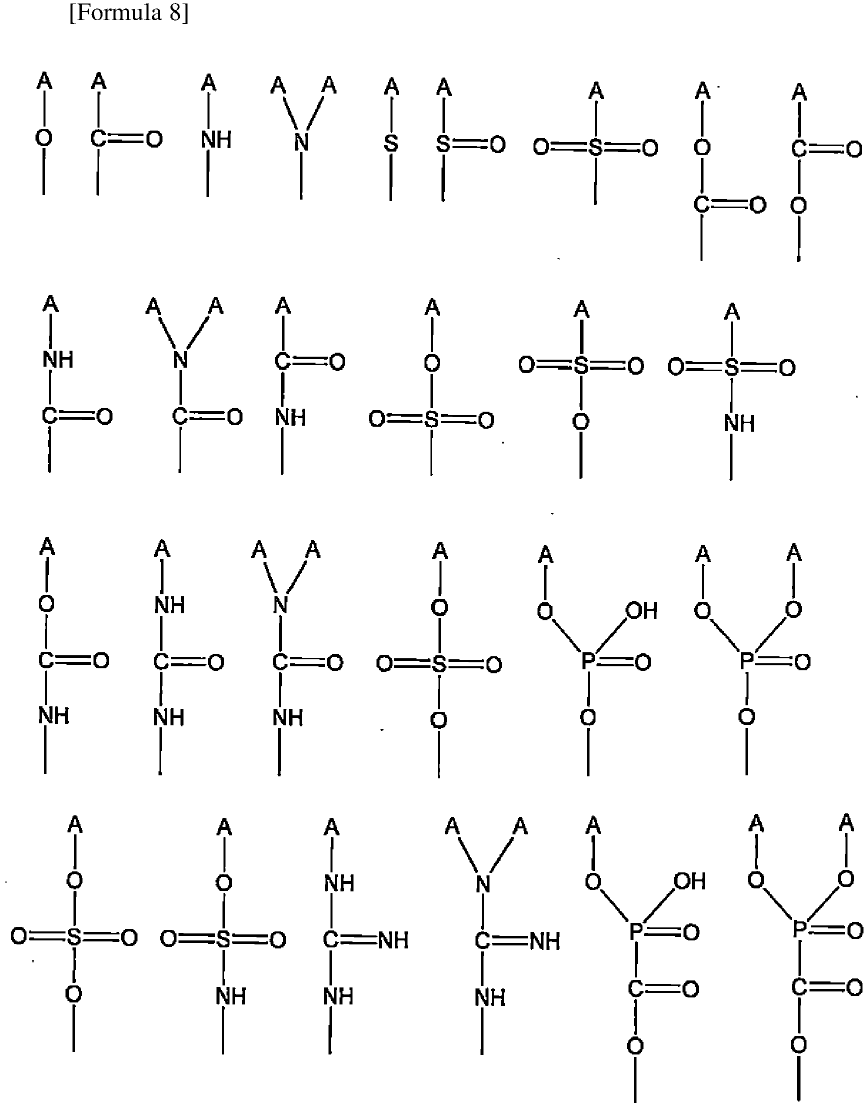

The number of carbon atoms of the hydrocarbon group (including its substituents, if any) is not particularly limited, but the upper limit thereof may be, for example, 20 or less, 15 or less, 10 or less, 8 or less, or 6 or less. The lower limit thereof depends on the type of the hydrocarbon group, but may be 1 or more for alkyl groups, 2 or more for alkenyl and alkynyl groups, and 3 or more, such as 4 or more, or 5 or more, for cycloalkyl groups. Examples of the number of atoms include, for example, 1, 2, 3, 4, 5, 6, 7, 8, 9, 10, 11, 12, 13, 14, 15, 16, 17, 18, 19, or 20.

The total number of carbon atoms and hetero atoms of the heterocyclic group (including its substituents, if any) is not particularly limited, but the upper limit thereof may be, for example, 20 or less, 15 or less, 10 or less, 8 or less, or 6 or less. The lower limit thereof depends on the type of the heterocyclic structure, but may typically be 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Among them, each of R²¹ and R²² may preferably be selected, independently of each other, from hydrogen atom, hydroxyl group, thiol group, carboxyl group, nitro group, cyano group, or halogen atom, or, that may have one or more substituents, amino group, alkyl group, alkenyl group, cycloalkyl group, alkoxy group, aryl group, aryloxy group, acyl group, heterocyclic group, and heterocyclic oxy group.

Specific examples of R²¹and R²² include, although are not limited to, the following.

*Hydrogen atom, hydroxyl group, thiol group, carboxyl group, nitro group, and cyano group;
*Halogen atoms such as fluorine atom, chlorine atom, bromine atom, and iodine atom;
*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Alkoxy groups such as methoxy group, ethoxy group, propoxy group, butoxy group, sec-butoxy group, and tert-butoxy group;
*Aryl groups such as phenyl group, benzyl group, tolyl group, naphthyl group, and anthracenyl group;
*Aryloxy groups such as phenyloxy group, benzyloxy group, and naphthyloxy group;
*Acyl groups such as acetyl group, propionyl group, benzoyl group, p-methoxybenzoyl group, and cinnamoyl group;
*Unsubstituted amino group and substitution amino groups such as dimethylamino group, benzylamino group, and triphenylmethylamino group;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, carbazolyl group; and
*Heterocyclic oxy groups such as furanyl oxy group, pyrrolyl oxy group, indolyl oxy group, and quinolyl oxy group.

Of the groups mentioned above, those having a carboxyl group may or may not have a protective group. When such a group with a carboxyl group has a protective group, the reaction selectivity with the carboxyl group on the right side of formula (R1) is usually superior to that with the carboxyl groups present on the other substituents, although it may also depend on the reactivity of the compound (R1) and the compound (R2) used in the reaction.

R²³ represents a hydrogen atom, carboxyl group, or hydroxyl group, or a monovalent hydrocarbon group or heterocyclic group which may optionally have one or more substituents. When any of these groups have one or more substituents, examples of such substituents are as explained above. Examples of the number of substituents are, e.g., 5, 4, 3, 2, 1, or 0.

When R²³ is a monovalent hydrocarbon group or heterocyclic group which may optionally have one or more substituents, a linking group may exist between the hydrocarbon group or heterocyclic group and the nitrogen atom to which it binds. Such linking groups may be selected from, although are not limited to, independently of each other, the following structures (in each formula below, A represents, independently of each other, a monovalent hydrocarbon group or heterocyclic group which may optionally have one or more substituents. When two or more As exist in a single group, they may be identical to each other or different from each other.).

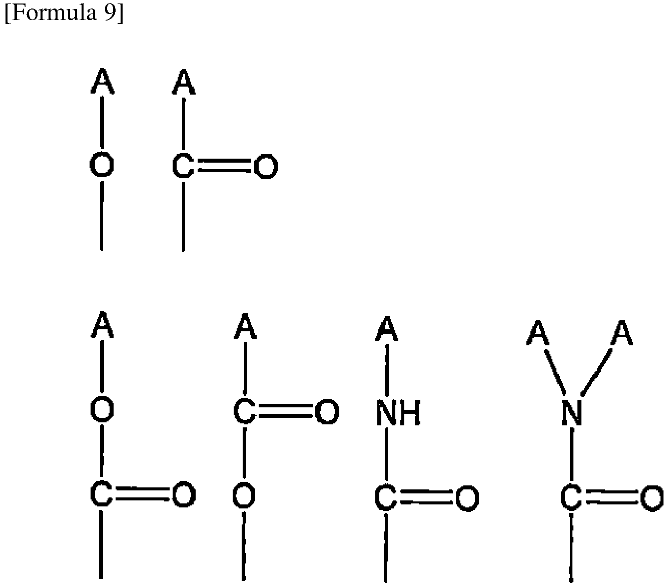

The number of carbon atoms of the hydrocarbon group (including its substituents, if any) is not particularly limited, but the upper limit thereof may be, for example, 20 or less, 15 or less, 10 or less, 8 or less, or 6 or less. The lower limit thereof depends on the type of the hydrocarbon group, but may be 1 or more for alkyl groups, 2 or more for alkenyl and alkynyl groups, and 3 or more, such as 4 or more, or 5 or more, for cycloalkyl groups. Examples of the number of atoms include, for example, 1, 2, 3, 4, 5, 6, 7, 8, 9, 10, 11, 12, 13, 14, 15, 16, 17, 18, 19, or 20.

The total number of carbon atoms and hetero atoms of the heterocyclic group (including its substituents, if any) is not particularly limited, but the upper limit thereof may be, for example, 20 or less, 15 or less, 10 or less, 8 or less, or 6 or less. The lower limit thereof depends on the type of the heterocyclic structure, but may typically be 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Among them, R²³ may preferably be selected, independently of each other, from hydrogen atom, hydroxyl group, or carboxyl group, or, that may have one or more substituents, alkyl group, alkenyl group, cycloalkyl group, alkoxy group, aryl group, aryloxy group, acyl group, heterocyclic group, and heterocyclic oxy group.

Specific examples of R²³ include, although are not limited to, the following.

*Hydrogen atom, hydroxyl group, and carboxyl group;
*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Aryl groups such as phenyl group, benzyl group, tolyl group, naphthyl group, anthracenyl group; and
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, and carbazolyl group.

R²¹ and R²³ may bind to each other and, along with R²¹ the carbon atom to which it binds and R²³ the nitrogen atom to which it binds, a heterocyclic ring which may optionally have one or more substituents. When any of these groups have one or more substituents, examples of such substituents are as explained above. Examples of the number of substituents are, e.g., 5, 4, 3, 2, 1, or 0s.

The total number of carbon atoms and hetero atoms of the heterocyclic group (including its substituents, if any) is not particularly limited, but the upper limit thereof may be, for example, 20 or less, 15 or less, 10 or less, 8 or less, or 6 or less. The lower limit thereof depends on the type of the heterocyclic structure, but may typically be 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Specific examples of such hetero rings include, but are not limited to, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, and 2,5-dihydro-1,3-thiazolyl group.

A¹¹, A¹², A²¹, and A²² represent, independently of each other, a bivalent aliphatic hydrocarbon group having 1 to 3 carbon atoms which may optionally have one or more substituents. Specific examples include, although are not limited to, methylene group, ethylene group, propylene group, and isopropylene group, as well as any groups derived from these groups via substitution with one or more substituents. Examples of the number of such substituents include, for example, 3, 2, 1, or 0.

p11, p12, p21, and p22 represent, independently of each other, 0 or 1.

n¹ represents an integer of 1 or higher, and defines the number of the structure units parenthesized with [ ] of formula (R1). When n¹ is 1, the compound (R1) is an amino acid, while when n¹ is 2 or more, the compound (R1) is a peptide. The upper limit for n¹ is not particularly limited so long as the amidation step proceeds, but may preferably be, for example, 100 or less, 80 or less, 60 or less, 50 or less, 40 or less, 30 or less, 20 or less, 15 or less, 12 or less, or 10 or less. Specific examples of n¹ include 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, and 100.

n² represents an integer of 1 or higher, and defines the number of the structure units parenthesized with [ ] of formula (R2) . When n² is 1, the compound (R2) is an amino acid, while when n² is 2 or more, the compound (R2) is a peptide. The upper limit for n² is not particularly limited so long as the amidation step proceeds, but may preferably be, for example, 1, 2, 3, 4, 5, 6, 7, 8, 9, 10. However, n² may preferably be 1. In other words, the compound (R2) may preferably be an amino acid.

Needless to say, when n² is 2 or more, then each of R²¹, R²², R²³, A²¹, A²², p21, and p22 in the structure parenthesized with [ ] may be either identical to each other or different from each other among the two or more amino acid residues. In other words, when the compound (R2) is a peptide, then the two or more amino acid residues constituting the peptide may be either identical to each other or different from each other.

PG^{b} represents a protective group for carboxyl groups. The protective group for carboxyl groups PG^{b} is not particularly limited so long as it can protect a carboxyl group during an amidation reaction, and can be deprotected after the reaction so as to restore the carboxyl group. Details of the protective group for carboxyl groups PG^{b} will be explained later, s

The amino group on the left side of formula (R2) in the compound (R2) may form a salt with an acid. In this case, examples of the acid include, but are not limited to' aliphatic carboxylic acids with 1 to 5 carbon atoms, such as acetic acid and propionic acid; and trifluoroacetic acid, hydrochloric acid, hydrobromic acid, sulfuric acid, nitric acid, phosphoric acid, boric acid, and sulfonic acid.

### *General matters for the substrate compounds

The combination of the substrate compounds (R1) and (R2) is not particularly limited, and can be selected and combined according to the structure of the peptide compound (P) to be finally synthesized. Each of the substrate compounds (R1) and (R2) may be, independently of each other, either a single compound used alone or two or more compounds in any combination and at any ratios.
s

Some or all of the substrate compound (R1) or (R2) may be linked or immobilized to a reactant, resin, or other carrier at any of their substituents. In this case, the type of the reactant, resin, or other carrier is not limited. Any known reactant, resin, or other carrier can be used as long as it does not substantially interfere with the amide reaction in the production method according to the present invention and does not depart from the purpose of the present invention. There are no restrictions the manner of linking and immobilizing the reactant compound to the reactant, resin, or other carrier, although it is preferable to form a covalent bond between any substituent of the reactant compound and any substituent present on the reactant, resin, or other carrier. There are also no restrictions on the type of each substituent and the method of forming a covalent bond. Any known substituent and covalent bond formation method can be used as long as it does not substantially interfere with the amide bond reaction in the production method according to the present invention and does not depart from the purpose of the present invention. When the reactant compounds are amino acids or peptides, such as in the case of Embodiment (3), the reactant compound may be linked and immobilized to a reactant, resin, or other carrier by covalent bonding using a carboxyl or amino group of the reactant compound (other than the carboxyl ester or amino group being the target of the amide reaction). Such a mode can be regarded as a variation of the embodiment in which the carboxyl or amino group of the reactant compound (other than the carboxyl ester or amino group being the target of the amide reaction) is protected by introducing a protective group.

### *Protective group for amino groups:

There are a wide variety of protective groups for amino groups known to the art. Examples thereof include monovalent hydrocarbon groups that may have one or more substituents and monovalent heterocyclic groups that may have one or more substituents. Preferred among these are monovalent hydrocarbon groups that may have one or more substituents. There may be any intervening linking group between the hydrocarbon group or heterocyclic group and the nitrogen atom of the amino group to be protected (the nitrogen atom in formula (3-1) to which PG¹ binds). Such a linking group is not particularly limited, but may be selected, independently of each other, from the linking groups listed below (where, in the chemical formulae below, A represents a monovalent hydrocarbon group or a heterocyclic group, each of which may independently have one or more substituents. When two A's are present in the same group, they may be identical to each other or different from each other.).

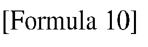

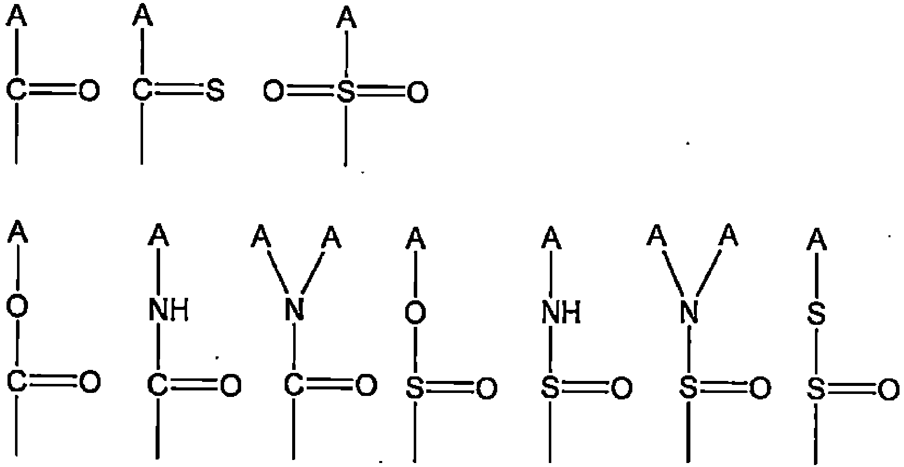

The number of carbon atoms in the protective group may typically be 1 or more, or 3 or more, and typically 20 or less, or 15 or less.

Among them, the amino-protective group may preferably be one or more selected from the group consisting of a monovalent hydrocarbon group, acyl group, hydrocarbon oxycarbonyl group, hydrocarbon sulfonyl group, and amide group that may have one or more substituents.

Specific examples of the amino-protective groups are listed below. Incidentally, an amino-protective group may be referred to either by the name of the functional group excluding the nitrogen atom of the amino group to which it binds or by the name of the group including the nitrogen atom to which it binds. The following list includes either or both of these names for each protective group.

Examples of unsubstituted or substituted hydrocarbon groups include: alkyl groups such as methyl group, ethyl group, and propyl group; alkenyl groups such as ethenyl group, propenyl group, and allyl group; alkynyl groups such as propargyl group; cycloalkyl groups such as cyclopropyl group, cyclobutyl group, cyclopentyl group, and cyclohexyl group; aryl groups such as phenyl group, benzyl group, p-methoxybenzyl group, tolyl group, and triphenylmethyl group (Troc group); and substituted hydrocarbon groups such as cyanomethyl group. The number of carbon atoms may typically be 1 or more, or 3 or more, and typically 20 or less, or 15 or less.

Specific examples of non-substituted or substituted acyl groups include: benzoyl group (Bz), orthomethoxybenzoyl group, 2,6-dimethoxybenzoyl group, paramethoxybenzoyl group (PMPCO), cinnamoyl group, and phthaloyl group (Phth).

Specific examples of non-substituted or substituted hydrocarbon oxycarbonyl groups include: tert-butoxy carbonyl (Boc) group, benzyloxycarbonyl (Cbz or Z) group, methoxy carbonyl group, ethoxy carbonyl group, 2-(trimethylsilyl)ethoxy carbonyl (Teoc) group, 2-phenyl ethoxy carbonyl group, 1-(1-adamantyl)-1-methyl ethoxy carbonyl group, 1-(3,5-di-t-butylphenyl)-1-methyl ethoxy carbonyl group, vinyloxycarbonyl group, allyoxycarbonyl (Alloc) group, N-hydroxy piperidinyl oxycarbonyl group, p-methoxybenzyloxycarbonyl group, p-nitrobenzyloxycarbonyl group, 2-(1,3-dithianyl)methoxy carbonyl, m-nitrophenoxycarbonyl group, 3,5-dimethoxybenzyloxycarbonyl group, o-nitrobenzyloxycarbonyl group, 2,2,2-trichloroethoxy carbonyl (Troc) group, 9-fluorenyl methyloxycarbonyl (Fmoc) group.

Specific examples of non-substituted or substituted hydrocarbon sulfonyl groups include: methanesulfonyl group (Ms), toluene sulfonyl group (Ts), and 2- or 4-nitrobenzene sulfonyl group (Ns).

Specific examples of unsubstituted or substituted amide groups include: acetamide, o-(benzoyloxymethyl)benzamide, 2-[(t-butyl-diphenyl-siloxy)methyl]benzamide, 2-toluenesulfonamide, 4-toluenesulfonamide, 2-nitro benzene sulfonamide, 4-nitro benzene sulfonamide, tert-butylsulfinylamide, 4-toluenesulfonamide, 2-(trimethylsilyl)ethanesulfonamide, and benzyl sulfonamide.

In terms of deprotection methods, the protective group may be deprotected by, e.g., at least one of the following methods: deprotection by hydrogenation, deprotection by weak acid, deprotection by fluorine ion, deprotection by one-electron oxidizing agent, deprotection by hydrazine, and deprotection by oxygen.

Preferred examples of the amino protective group include: mesyl group (Ms), tert-butoxycarbonyl group (Boc), benzyl group (Bn or Bzl), benzyloxycarbonyl group (Cbz), benzoyl group (Bz), p-methoxybenzyl group (PMB), 2,2,2-trichloroethoxycarbonyl group (Troc), allyloxycarbonyl group (Alloc), 2,4-dinitrophenyl group (2,4-DNP), phthaloyl group (Phth), p-methoxybenzoyl group (PMPCO), cinnamoyl group, toluenesulfonyl group (Ts), 2- or 4-nitrobenzenesulfonyl group (Ns), cyanomethyl group, 9-fluorenylmethyloxycarbonyl group (Fmoc), and 2-(trimethylsilyl)ethoxy carbonyl (Teoc) group. These protective groups are preferred because, as mentioned above, they can easily protect the amino group and can be removed under relatively mild conditions.

More preferable examples of the amino protective group include: mesyl group (Ms), tert-butoxycarbonyl group (Boc), benzyloxycarbonyl group (Cbz), benzyl group (Bn), p-methoxybenzyl group (PMB), 2,2,2-trichloroethoxycarbonyl group (Troc), allyloxycarbonyl group (Alloc), p-methoxybenzoyl group (PMPCO), benzoyl group (Bz), cyanomethyl group, cinnamoyl group, 2- or 4-nitrobenzenesulfonyl group (Ns), toluenesulfonyl group (Ts), phthaloyl group (Phth), 2,4-dinitrophenyl group (2,4-DNP), 9-fluorenylmethyloxycarbonyl group (Fmoc), and 2-(trimethylsilyl)ethoxy carbonyl (Teoc) group.

### *Protective group for carboxyl groups:

Examples of carboxyl protective groups include monovalent hydrocarbon groups or heterocyclic groups that may have one or more substituents. The details of the substituents, if any, are described above. Examples of the number of such substituents include, for example, 5, 4, 3, 2, 1, or 0.

The upper limit of the number of carbon atoms of each hydrocarbon group (including its substituents, if any) may be for example 20 or less, 15 or less, 10 or less, 8 or less, or 6 or less. The lower limit thereof depends on the type of the hydrocarbon group, but may be 1 or more for alkyl groups, 2 or more for alkenyl and alkynyl groups, and 3 or more, such as 4 or more, or 5 or more, for cycloalkyl groups. Examples of the number of atoms include, for example, 1, 2, 3, 4, 5, 6, 7, 8, 9, 10, 11, 12, 13, 14, 15, 16, 17, 18, 19, or 20.

The upper limit of the total number of carbon atoms and hetero atoms of each heterocyclic group (including its substituents, if any) may be, for example, 20 or less, 15 or less, 10 or less, 8 or less, or 6 or less. The lower limit thereof depends on the type of the heterocyclic structure, but may typically be 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Examples of the carboxyl protective group include, but are not limited to, the following.

*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Aryl groups such as phenyl group, benzyl group, tolyl group, naphthyl group, anthracenyl group;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, and carbazolyl group; and
*Silicon-based protective groups such as trimethylsilyl (TMS) group, triethylsilyl (TES) group, triisopropylsilyl (TIPS) group, tri(tert-butyl)silyl (TBS) group, tert-butyldiphenylsilyl (TBDPS) group and tris(trialkylsilyl)silyl group.

### *Silane compounds:

In the production method of the present invention (1), a silane compound may coexist in the reaction system. Carrying out the reaction in the presence of a silane compound in the reaction system may result in various advantages, such as increased reaction yield and improved stereoselectivity.

Examples of silane compounds include: various tris{halo(preferably fluorine)-substituted alkyl}silanes such as HSi(OCH(CF₃)₂)₃, HSi(OCH₂CF₃)₃, HSi(OCH₂CF₂CF₂H)₃, and HSi(OCH₂CF₂CF₂CF₂CF₂H)₃; and trifluoromethane sulfonic acid trimethylsilyl (TMS-OTf), 1-(trimethylsilyl)imidazole (TMSIM), dimethyl ethylsilylimidazole (DMESI), dimethyl isopropylsilylimidazole (DMIPSI), 1-(tert-butyl dimethylsilyl)imidazole (TBSIM), 1-(trimethylsilyl)triazole, 1-(tert-butyl dimethylsilyl)triazole, dimethylsilylimidazole, dimethylsilyl (2-methyl)imidazole, trimethyl bromosilane (TMBS), trimethyl chlorosilane (TMCS), N-methyl-N-trimethylsilyl trifluoroacetamide (MSTFA), N,O-bis(trimethylsilyl)trifluoroacetamide (BSTFA), N,O-bis(trimethylsilyl)acetamide (BSA), N-(trimethylsilyl)dimethylamine (TMSDMA), N-(tertbutyldimethylsilyl)-N-methyl trifluoroacetamide (MTBSTFA), and hexamethyldisilazane (HMDS). These may be used either any one singly or any two or more in any combination at any ratios.

### *Lewis acid catalysts:

In the production method of the present invention (1), a Lewis acid catalyst may coexist in the reaction system. Carrying out the reaction in the presence of a Lewis acid in the reaction system may result in various advantages, such as increased reaction yield and improved stereoselectivity. However, if a Lewis acid catalyst is used, it may be necessary to separate and remove the Lewis acid catalyst from the reaction product. Therefore, it is preferable to determine whether or not to use a Lewis acid catalyst in consideration of the purpose of using the production method according to the present invention.

When a Lewis acid catalyst is used in the production method according to the present invention, the type of catalyst is not limited, but it may preferably be a metal compound that functions as a Lewis acid. Examples of metal elements constituting the metal compound include various metals belonging to groups 2 through 15 of the periodic table. Examples of such metal elements include boron, magnesium, gallium, indium, silicon, calcium, lead, bismuth, mercury, transition metals, and lanthanoid elements. Examples of transition metals include scandium, titanium, vanadium, chromium, manganese, iron, cobalt, nickel, copper, zinc, yttrium, zirconium, niobium, molybdenum, technetium, ruthenium, rhodium, palladium, tin, silver, cadmium, hafnium, tantalum, tungsten, rhenium, osmium, iridium, platinum, gold, and thallium. Examples of lanthanoid elements include lanthanum, cerium, neodymium, samarium, europium, gadolinium, holmium, erbium, thulium, ytterbium. From the viewpoint of excellent reaction acceleration and highly stereoselective production of amide compounds, the metal element may preferably be one or more selected from titanium, zirconium, hafnium, tantalum, niobium, boron, vanadium, tungsten, neodymium, iron, lead, cobalt, copper, silver, palladium, tin, and thallium, more preferably one or more selected from titanium, zirconium, hafnium, tantalum, and niobium. The metal compound may contain one, two or more metal atoms. If the metal compound contains two or more metal atoms, the two or more metal atoms may be either of the same metal element or of different metal elements.

Ligands constituting the metal compound may be selected according to the type of the metal element. Examples of ligands include: substituted or unsubstituted linear- or branched-chain alkoxy groups containing 1 to 10 carbon atoms, such as methoxy group, ethoxy group, propoxy group, butoxy group, trifluoroethoxy group, and trichloroethoxy group; halogen atoms such as fluorine atom, chlorine atom, bromine atom, iodine atom; aryloxy groups having 1 to 10 carbon atoms; acetylacetonate group (acac), acetoxy group (AcO), trifluoromethane sullfonate group (TfO); substituted or unsubstituted linear- or branched-chain alkyl groups having 1 to 10 carbon atoms; phenyl group, oxygen atom, sulfur atom, group -SR (where R represents a substituent exemplified by substituted or unsubstituted hydrocarbon groups containing 1 to 20 carbon atoms), group -NRR' (where R and R', independently of each other, represent a hydrogen atom or a substituent exemplified by substituted or unsubstituted hydrocarbon groups containing 1 to 20 carbon atoms), and cyclopentadienyl (Cp) group.

Preferred metal compounds among these are titanium compounds, zirconium compounds, hafnium compounds, tantalum compounds, or niobium compounds. Examples of these metal compounds are indicated below. These may be used either any one singly or any two or more in any combination at any ratios.

Examples of titanium compounds include those represented by TiX¹₄ (where 4 X¹'s, independently of each other, represent any of the ligands exemplified above, provided that 4 X¹'s may be the same type of ligand or different from each other.). When X¹ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X¹ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X¹ is a halogen atom, it may preferably be a chlorine atom or a bromine atom. Preferred examples of titanium compounds include Ti(OMe)₄, Ti(OEt)₄, Ti(OPr)₄, Ti(Oi-Pr)₄, Ti(OBu)₄, Ti(Ot-Bu)₄, Ti(OCH₂CH(Et)Bu)₄, CpTiCl₃, Cp₂TiCl₂, Cp₂Ti(OTf)₂, (i-PrO)₂TiCl₂, and (i-PrO)₃TiCl.

Examples of zirconium compounds include those represented by ZrX²₄ (where 4 X²'s, independently of each other, represent any of the ligands exemplified above, provided that 4 X²'s may be the same type of ligand or different from each other.). When X² is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X² is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X² is a halogen atom, it may preferably be a chlorine atom or a bromine atom. Preferred examples of zirconium compounds include Zr(OMe)₄, Zr(OEt)₄, Zr(OPr)₄, Zr(Oi-Pr)₄, Zr(OBu)₄, Zr(Ot-Bu)₄, Zr(OCH₂CH(Et)Bu)₄, CpZrCl₃, Cp₂ZrCl₂, Cp₂Zr(OTf)₂, (i-PrO)₂ZrCl₂, and (i-PrO)₃ZrCl.

Examples of hafnium compounds include those represented by HfX³₄ (where 4 X³'s, independently of each other, represent any of the ligands exemplified above, provided that 4 X³'s may be the same type of ligand or different from each other.). When X³ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X³ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X³ is a halogen atom, it may preferably be a chlorine atom or a bromine atom. Preferred examples of hafnium compounds include HfCp₂Cl₂, HfCpCl₃, and HfCl₄.

Examples of tantalum compounds include those represented by TaX⁴₅ (where 5 X⁴'s, independently of each other, represent any of the ligands exemplified above, provided that 5 X⁴'s may be the same type of ligand or different from each other.). When X⁴ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X⁴ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X⁴ is a halogen atom, it may preferably be a chlorine atom or a bromine atom. Preferred examples of tantalum compounds include tantalum alkoxide compounds (e.g., compounds in which X⁴ is an alkoxy group) such as Ta(OMe)₅, Ta(OEt)₅, Ta(OBu)s, Ta(NMe₂)₅, Ta(acac)(OEt)₄, TaCl₅, TaCl₄(THF), and TaBr₅. Other examples are those in which X⁴ is an oxygen, such as TazOs.

Examples of niobium compounds include those represented by NbX⁵₅ (where 5 X⁵'s, independently of each other, represent any of the ligands exemplified above, provided that 5 X⁵'s may be the same type of ligand or different from each other.). When X⁵ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X⁵ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X⁵ is a halogen atom, it may preferably be a chlorine atom or a bromine atom. Preferred examples of niobium compounds include niobium alkoxide compounds (e.g., compounds in which X⁵ is an alkoxy group) such as NbCl₄(THF), NbCl₅, Nb(OMe)s, and Nb(OEt)s. Other examples are those in which X⁵ is an oxygen, such as NbzOs.

The Lewis acid catalyst may be loaded on a carrier. There are no particular restrictions on the carrier on which the Lewis acid catalyst is to be loaded, and any known carrier can be used. Also, any known method can be used to load the Lewis acid catalyst on the carrier.

### *Other ingredients:

In the production method of the present invention (1), the reaction system may contain one or more other ingredients, in addition to the amino-protected lactam compound of the formula (R1) and the amino acid ester or peptide ester compound of formula (R2) as the substrate compounds, as well as the optionally-used silane compound and/or Lewis acid catalyst. Examples of such other ingredients include, although are not limited to, conventional catalysts (other than Lewis acid catalysts) for amidation reaction, bases, phosphorus compounds, and solvents. These may be used either any one singly or any two or more in any combination at any ratios.

Examples of catalysts (other than Lewis acid catalysts) include methyl aluminum bis(4-bromo-2,6-di-tert-butyl phenoxide) (MABR), trifluoromethane sulfonic acid trimethylsilyl (TMS-OTf), and methyl aluminum bis(2,6-di-tert-butyl phenoxide) (MAD). These may be used either any one singly or any two or more in any combination at any ratios.

Any known bases can be used without any limitation, so long as they are known to improve reaction efficiency. Examples of bases include: amines having 1 to 4 straight chain or branched chain alkyl groups with 1 to 10 carbon atoms, such as tetrabutyl ammonium fluoride (TBAF), triethylamine (Et₃N), diisopropylamine (i-Pr₂NH), and diisopropyl ethylamine (i-Pr₂EtN); and inorganic bases such as fluoride cesium. These may be used either any one singly or any two or more in any combination at any ratios.

Examples of phosphorus compounds include: phosphine compounds such as trimethylphosphine, triethylphosphine, tripropylphosphine, trimethyloxyphosphine, triethyloxyphosphine, tripropyloxyphosphine, triphenylphosphine, trinaphthylphosphine, triphenyloxyphosphine, tris(4-methylphenyl)phosphine, tris(4-methoxyphenyl)phosphine, tris(4-fluorophenyl)phosphine, tris(4-methylphenyloxy)phosphine, tris(4-methoxyphenyloxy)phosphine, tris(4-fluorophenyloxy)phosphine; phosphate compounds such as trimethylphosphate, triethylphosphate, tripropylphosphate, trimethyloxyphosphate, triethyloxyphosphate, tripropyloxyphosphate, triphenylphosphate, trinaphthylphosphate, triphenyloxyphosphate, tris(4-methylphenyl)phosphate, tris(4-methoxyphenyl)phosphate, tris(4-fluorophenyl)phosphate, tris(4-methylphenyloxy)phosphate, tris(4-methoxyphenyloxy)phosphate, and tris(4-fluorophenyloxy)phosphate; and multi-valence phosphine compounds or multi-valence phosphate compounds such as 2,2'-bis(diphenylphosphino)-1,1'-binaphthyl (BINAP) and 5,5'-bis(diphenylphosphino)-4,4'-bi-1,3-benzodioxole (SEGPHOS). These may be used either any one singly or any two or more in any combination at any ratios.

From the viewpoint of increasing the reaction efficiency, a solvent may be used during the reaction. Solvents are not particularly restricted, but may include aqueous and organic solvents. Examples of organic solvents include, although are not limited to: aromatic hydrocarbons such as toluene and xylene; pentane; ethers such as petroleum ether, tetrahydrofuran (THF), 1-methyl tetrahydrofuran (1-MeTHF), diisopropyl ether (i-Pr₂O), diethyl ether (Et₂O), and cyclopentyl methyl ether (CPME); nitrogen-containing organic solvents such as acetonitrile (MeCN); chloride-containing organic solvents such as dichloromethane (DCM); esters such as ethyl acetate (AcOEt); and organic acids such as acetic acid. These solvents may be used either any one singly or any two or more in any combination at any ratios.

### *Reaction procedure:

The production method of the present invention (1) may be carried out by mixing the amino-protected lactam compound of the formula (R1) and the amino acid ester or peptide ester compound of formula (R2) as the substrate compounds to cause reaction. When any of silane compounds, Lewis acid catalysts, and/or other ingredients (such as catalysts other than Lewis acid catalysts, bases, and/or phosphorus compounds) are used as optional ingredients, they may be mixed with the substrate compounds and optional ingredients mentioned above. When a solvent is used as an optional ingredient, the above ingredients may be added into the solvent and mixed in the solvent.

For any of these ingredients, the entire amount may be added to the reaction system all at once, may be divided into several portions, or may continuously be added to the reaction system in small quantities.

### *Usage ratio of each component:

The amount of each component used in the production method (1) of the present invention is not limited, but may preferably be as follows.

The amount ratio of the amino-protected lactam compound of formula (R1) to the amino acid ester or peptide ester compound of formula (R2) is not particularly limited, but per mole of the compound of formula (R1), the compound of formula (R2) may be used in an amount range of, for example, 0.1 moles or more, or 0.2 moles or more, or 0.3 moles or more, or 0.4 moles or more, or 0.5 moles or more, and 20 moles or less, or 10 moles or less, or 5 moles or less, or 4 moles or less, or 3 moles or less. It is preferable to use the compound of formula (R2) in a higher amount than that of the compound of formula (R1) in order to increase the reaction efficiency. Specifically, about 2 moles of compound of formula (R2) can be used per mole of the compound of formula (R1). Needless to say, it is necessary to use at least one mole each of the compound of formula (R1) and the compound of formula (R2) as substrates for the target production quantity of the compound of formula (P) to be produced.

When a silane compound is used, the amount of the silane compound is not particularly limited, but when the amount of the compound of formula (R1) is 100 mol%, the silane compound may be used in an amount range of, for example 0.1 mol % or more, or 0.2 mol % or more, or 0.3 mol % or more, and 50 mol % or less, or 30 mol % or less, or 20 mol % or less, or 15 mol % or less.

When a Lewis acid catalyst is used, the amount of the Lewis acid catalyst is not particularly limited, but when the amount of the compound of formula (R1) is 100 mol%, the Lewis acid catalyst may be used in an amount range of, for example, 0.1 mol % or more, or 0.2 mol % or more, or 0.3 mol % or more, and 50 mol % or less, or 30 mol % or less, or 20 mol % or less, or 15 mol % less.

When other optional ingredients are used, the amounts of such optional ingredients used may be adjusted as appropriate by referring to prior findings, such as those described in the present inventors' previous patents (Patent Literatures 1 to 6).

### *Reaction conditions:

The reaction conditions in the production method (1) of the present invention are not limited as long as the reaction proceeds, but examples are as follows.

The reaction temperature is not limited as long as the reaction proceeds, but may be within a range of for example 0 °C or more, or 10 °C or more, or 20 °C or more, and 100 °C or less, or 80 °C or less, or 60 °C or less.

The reaction pressure is also not limited as long as the reaction proceeds, and may be reduced pressure, normal pressure, or increased pressure. However, normal pressure may typically be adopted.

The reaction atmosphere is also not limited as long as the reaction proceeds, but may be an atmosphere with an inert gas such as argon or nitrogen.

The reaction time is also not limited as long as the reaction proceeds, but from the viewpoint of ensuring that the reaction proceeds fully and efficiently, it may be within a range of, for example, 10 minutes or more, or 20 minutes or more, or 30 minutes or more, and 80 hours or less, or 60 hours or less, or 50 hours or less.

The manufacturing method (1) may be carried out by a sequential method (batch method) or by a continuous method (flow method). Details of the specific implementation procedures for a sequential method (batch method) or a continuous method (flow method) are well known in the art.

### *Post-processing, etc. (e.g., purification and recovery):

Various post-treatments may be further applied to the peptide compound (P) obtained by the production method (1) of the present invention. For example, the generated peptide compound (P) may be isolated and purified according to conventional methods such as column chromatography, recrystallisation, etc. If the generated peptide compound (P) has an amino group and/or a carboxyl group protected by a protecting group, deprotection may be carried out according to the methods described below. The generated peptide compound (P) may be, either directly or after isolation and purification, subjected to the later stage process of the manufacturing method (2) of the present invention described below to produce a polypeptide with further elongated amino acid residues.

### [V. Others]

Various post-treatments may be further applied to the polypeptide compound of formula (P) obtained by the production method described above.

For example, the polypeptide compound of formula (P) obtained by the above-mentioned production method may be isolated and purified according to conventional methods such as column chromatography, recrystallisation, etc.

The polypeptide compound of formula (P) obtained by the production method described above may also be subjected to deprotection of the amino group protected by the protecting group. The method of deprotection of the protective amino group is not restricted, and various methods may be used depending on the type of protective group. Examples include deprotection by hydrogenation, deprotection by weak acids, deprotection by fluorine ions, deprotection by one-electron oxidants, deprotection by hydrazine, and deprotection by oxygen. The deprotection by hydrogenation may be carried out by, e.g.; (a) a method of causing deprotection in the presence of hydrogen gas using a metal catalyst such as palladium, palladium-carbon, palladium hydroxide, palladium-carbon hydroxide, etc., as a reduction catalyst; and (b) a method of causing deprotection in the presence of a metal catalyst such as palladium, palladium-carbon, palladium hydroxide, palladium-carbon hydroxide, etc., using a hydrotreating reductant such as sodium borohydride, lithium aluminum hydride, lithium borohydride, diborane, etc.

The polypeptide compound of formula (P) obtained by the production method described above may also be subjected to deprotection of the carboxyl group protected by the protecting group. The method of deprotection of the protective carboxyl group is not particularly restricted, and various methods can be used depending on the type of protective group. Examples include deprotection by hydrogenation, deprotection with bases, and deprotection with weak acids. Examples of deprotection methods with bases include methods such as deprotection using a strong base such as lithium hydroxide, sodium hydroxide or potassium hydroxide.

The polypeptide compound of formula (P) obtained by the above-mentioned production method (after deprotection and/or conversion of substituents, etc., if necessary) may be further used as a peptide compound of formula (R1) described above and again subjected to method (1) or (2). Alternatively, the polypeptide compound of formula (P) obtained by the production method described above may be subjected (after deprotection and/or substituent conversion or other treatment as necessary) to other conventionally known amidation or peptide production methods. Thus, a polypeptide compound of formula (P) may be linked to another amino acid or peptide by an amide bond to thereby elongate the amino acid residue and synthesize a larger polypeptide. By sequentially repeating these steps, it is in principle possible to synthesize a polypeptide with any number of amino acid residues and having any amino acid sequence.

The present inventors have filed the following prior patent applications relating to amidation reactions for linking amino acids or peptides and methods for producing polypeptides using such amidation reactions. It is also possible to carry out the production method of polypeptides according to the present invention in combination with any of the amidation reactions and polypeptide production methods described in these prior patent applications as appropriate and/or to modify the production method of polypeptides according to the present invention as appropriate, taking into account the conditions of the amidation reactions and polypeptide production methods described in these prior patent applications. The descriptions of these prior patent applications are incorporated herein by reference in their entirety.
(1) WO2017/204144 A (Patent Literature 1 mentioned above)
(2) WO2018/199146 A (Patent Literature 2 mentioned above)
(3) WO2018/199147 A (Patent Literature 3 mentioned above)
(4) WO2019/208731 A (Patent Literature 4 mentioned above)
(5) WO2021/085635 A (Patent Literature 5 mentioned above)
(6) WO2021/085636 A (Patent Literature 6 mentioned above)
(7) WO2021/149814 A (Patent Literature 7 mentioned above)

### EXAMPLES

The present invention will be described in more detail below with reference to examples. However, the present invention should in no way be bound by the following examples, and can be implemented in any form within the scope that does not depart from the purpose of the invention. The percentage values in the following description are expressed as mass percentages unless otherwise stated. Yields are determined by GC (gas chromatography) analysis using octane as an internal standard, or by isolation using chromatography. Diastereoselectivity of the products was determined by ¹H NMR analysis. The products were identified by ¹H NMR analysis and liquid chromatogram mass spectrometry (LC/MS).

### [Example Group I: Peptide synthesis using amino-protected lactams and amino acid esters at various ratios]

### (1) Example I-1: Synthesis of a peptide (Boc-6-HoGly-L-Ala-OtBu) using an amino-protected lactam (N-Boc-2-piperidone) with an amino acid ester (H-L-Ala-OtBu) at a molar ratio of 1:1

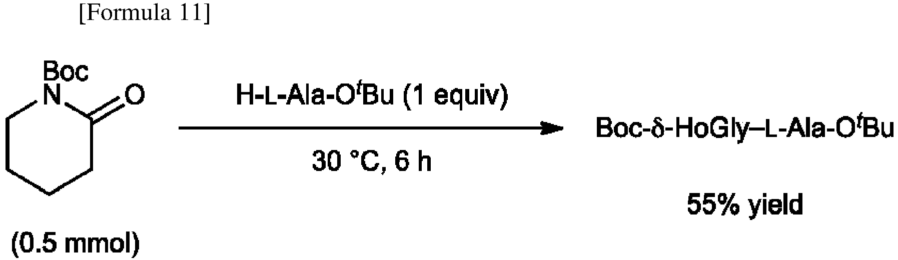

A 5.0 mL screw-cap vial equipped with a stirrer bar (Sm-Co) was charged with N-Boc-2-piperidone (N-Boc-δ-lactam) (99.625 mg, 0.50 mmol) and H-L-Ala-OtBu (72.6 mg, 0.50 mmol), and sealed with a screw cap. The mixture was caused to react with agitation at 30 °C for 6 hours. The reaction mixture was diluted with chloroform (CHCl₃) (3.0mL), and transferred to a silica (SiO₂) gel column with a pipette. The vial and the pipette used was washed with chloroform (12mL), and the wash liquid was added to the reaction mixture. The resulting mixture was purified with flash column chromatography (50 to 100 % AcOEt/hexane) to thereby produce the title compound as colorless and transparent liquid (yield: 55%, >99:1 er).

### (2) Example I-2: Synthesis of a peptide (Boc-δ-HoGly-L-Ala-OtBu) using an amino-protected lactam (N-Boc-2-piperidone) with an amino acid ester (H-L-Ala-OtBu) at a molar ratio of 1:2

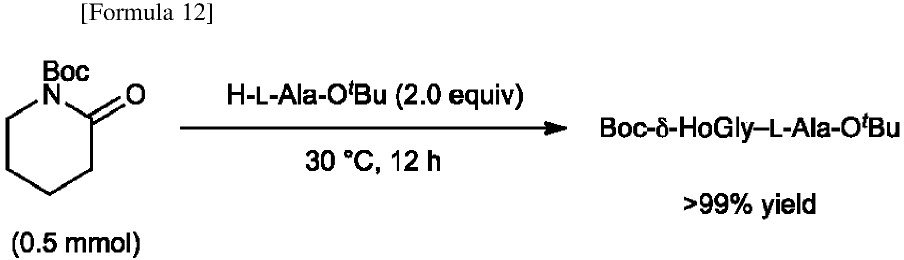

The same reaction procedure as Example I-1 was carried out except that the amount of H-L-Ala-OtBu used was changed to 145.2 mg (1.0 mmol), and the reaction time was changed to 12 hours, to thereby produce the title compound as colorless and transparent liquid (yield: >99%, >99:1 er).

### (3) Example I-3: Synthesis of a peptide (Boc-6-HoGly-L-Ala-OtBu) using an amino-protected lactam (N-Boc-2-piperidone) with an amino acid ester (H-L-Ala-OtBu) at a molar ratio of 2:1

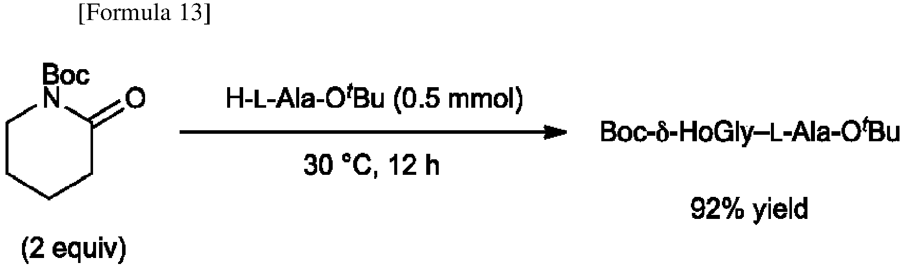

The same reaction procedure as Example I-1 was carried out except that the amount of N-Boc-2-piperidone used was changed to 199.25 mg (1.0 mmol), and the reaction time was changed to 12 hours, to thereby produce the title compound as colorless and transparent liquid (yield: 92%, >99:1 er).

### [Example Group II: Peptide synthesis by reacting amino-protected lactams and amino acid esters in various solvents]

### (1) Example II-1: Synthesis of a peptide (Boc-ð-HoGly-L-Ala-OtBu) by reacting an amino-protected lactam (N-Boc-2-piperidone) with an amino acid ester (H-L-Ala-OtBu) in AcOEt

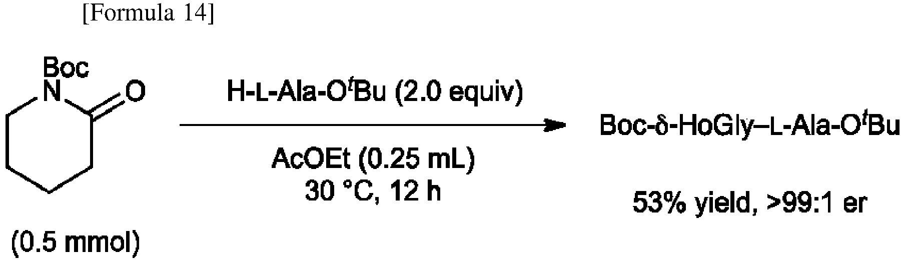

The same reaction procedure as Example I-1 was carried out except that the amount of H-L-Ala-OtBu used was changed to 145.2mg (1.0 mmol), 0.25mL of ethyl acetate (AcOEt) was added to the vial as a reaction solvent, and the reaction time was changed to 12 hours, to thereby produce the title compound as colorless and transparent liquid (yield: 53%, >99:1 er).

### (2) Example II-2: Synthesis of a peptide (Boc-ð-HoGly-L-Ala-OtBu) by reacting an amino-protected lactam (N-Boc-2-piperidone) with an amino acid ester (H-L-Ala-OtBu) in CPME

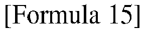

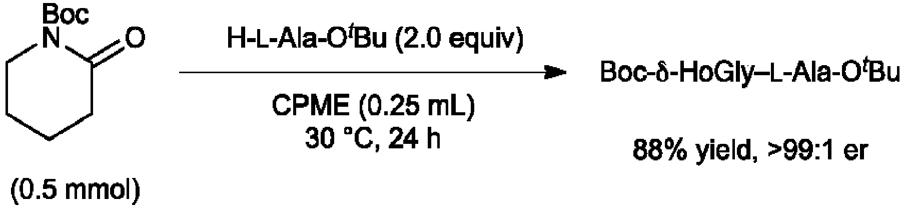

The same reaction procedure as Example II-1 was carried out except that the reaction solvent was changed to cyclopentyl methyl ether (CPME) 0.25mL, and the reaction time was changed to 24 hours, to thereby produce the title compound as colorless and transparent liquid (yield: 88%, >99:1 er).

### (3) Example II-3: Synthesis of a peptide (Boc-ð-HoGly-L-Ala-OtBu) by reacting an amino-protected lactam (N-Boc-2-piperidone) with an amino acid ester (H-L-Ala-OtBu) in DME

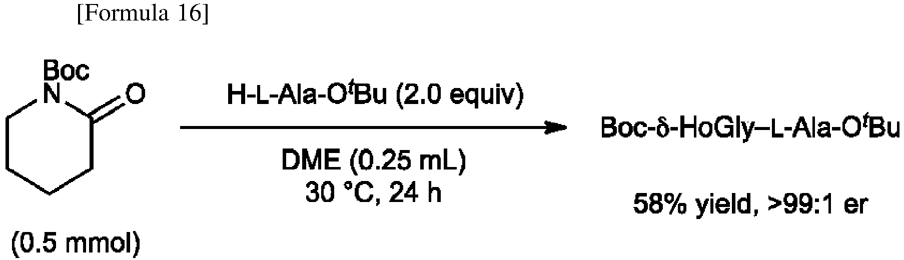

The same reaction procedure as Example II-1 was carried out except that the reaction solvent was changed to 0.25 mL of 1,2-dimethoxy ethane (DME), and the reaction time was changed to 24 hours, to thereby produce the title compound as colorless and transparent liquid (yield: 58%, >99:1 er).

### (4) Example II-4: Synthesis of a peptide (Boc-6-HoGly-L-Ala-OtBu) by reacting an amino-protected lactam (N-Boc-2-piperidone) with an amino acid ester (H-L-Ala-OtBu) in DMF

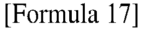

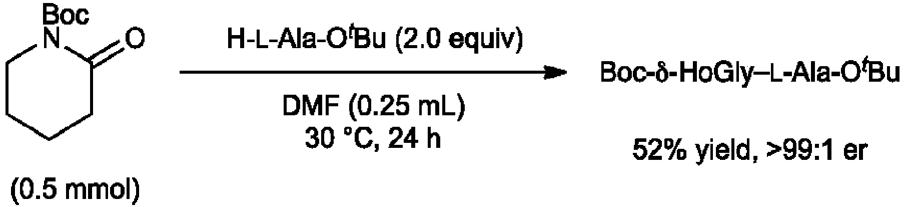

The same reaction procedure as Example II-1 was carried out except that the reaction solvent was changed to 0.25 mL of N,N-dimethyl formamide (DMF), and the reaction time was changed to 24 hours, to thereby produce the title compound as colorless and transparent liquid (yield: 52%, >99:1 er).

### (5) Example II-5: Synthesis of a peptide (Boc-6-HoGly-L-Ala-OtBu) by reacting an amino-protected lactam (N-Boc-2-piperidone) with an amino acid ester (H-L-Ala-OtBu) in tBuOH

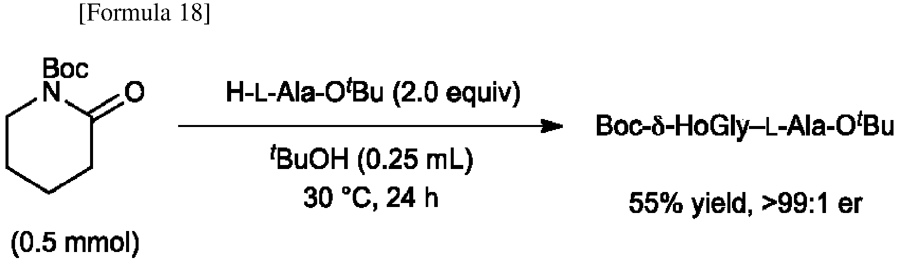

The same reaction procedure as Example II-1 was carried out except that the reaction solvent was changed to 0.25 mL of tert-butyl alcohol (tBuOH), and the reaction time was changed to 24 hours, to thereby produce the title compound as colorless and transparent liquid (yield: 55%, >99:1 er).

### (6) Example 11-6: Synthesis of a peptide (Boc-6-HoGly-L-Ala-OtBu) by reacting an amino-protected lactam (N-Boc-2-piperidone) with an amino acid ester (H-L-Ala-OtBu) in iPr₂OH

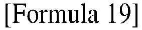

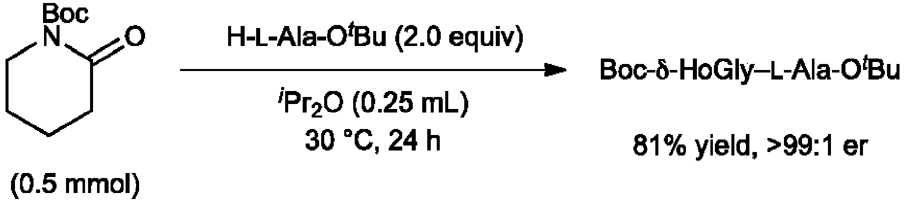

The same reaction procedure as Example II-1 was carried out except that the reaction solvent was changed to 0.25 mL of diisopropyl ether (iPr₂O), and the reaction time was changed to 24 hours, to thereby produce the title compound as colorless and transparent liquid (yield: 81%, >99:1 er).

### (7) Example II-7: Synthesis of a peptide (Boc-6-HoGly-L-Ala-OtBu) by reacting an amino-protected lactam (N-Boc-2-piperidone) with an amino acid ester (H-L-Ala-OtBu) in 2-Me-THF

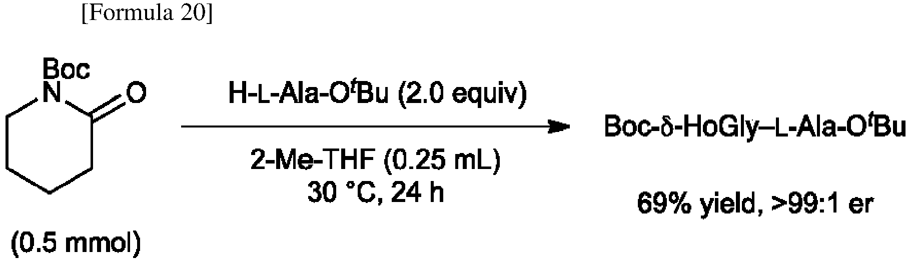

The same reaction procedure as Example II-1 was carried out except that the reaction solvent was changed to 0.25 mL of 2-methyl tetrahydrofuran (2-Me-THF), and the reaction time was changed to 24 hours, to thereby produce the title compound as colorless and transparent liquid (yield: 69%, >99:1 er).

### (8) Example 11-8: Synthesis of a peptide (Boc-6-HoGly-L-Ala-OtBu) by reacting an amino-protected lactam (N-Boc-2-piperidone) with an amino acid ester (H-L-Ala-OtBu) in TBME

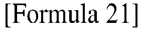

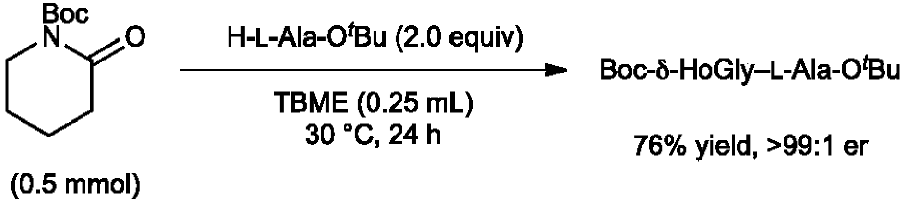

The same reaction procedure as Example II-1 was carried out except that the reaction solvent was changed to 0.25 mL of tert-butyl methyl ether (TBME), and the reaction time was changed to 24 hours, to thereby produce the title compound as colorless and transparent liquid (yield: 76%, >99:1 er).

### (9) Example 11-9: Synthesis of a peptide (Boc-6-HoGly-L-Ala-OtBu) by reacting an amino-protected lactam (N-Boc-2-piperidone) with an amino acid ester (H-L-Ala-OtBu) in toluene

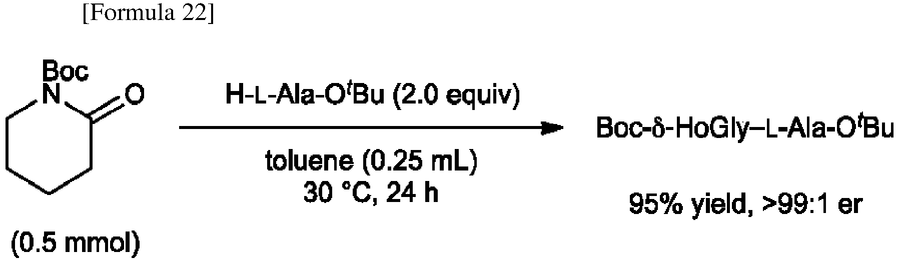

The same reaction procedure as Example II-1 was carried out except that the reaction solvent was changed to 0.25 mL of toluene, and the reaction time was changed to 24 hours, to thereby produce the title compound as colorless and transparent liquid (yield: 95%, >99:1 er).

### [Example Group III: Peptide synthesis by reacting amino-protected lactams and amino acid esters having various structures]

### (1) Example III-1: Synthesis of a peptide (Boc-β-HoGly-L-Ala-OtBu) by reacting an amino-protected 4-membered ring lactam (N-Boc-2-azetidinone) with an amino acid ester (H-L-Ala-OtBu)

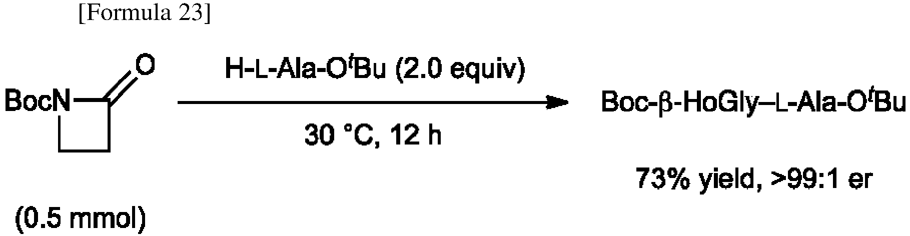

The same reaction procedure as Example I-1 was carried out except that 4-membered ring lactam N-Boc-2-azetidinone (N-Boc-β-lactam) (85.595 mg, 0.5 mmol) as used as an amino-protected lactam, the amount of H-L-Ala-OtBu used was changed to 145.2mg (1.0 mmol), and the reaction time was changed to 12 hours, to thereby produce the title compound as colorless and transparent liquid (yield: 73%, >99:1 er).

### (2) Example III-2: Synthesis of a peptide (Boc-γ-HoGly-L-Ala-OtBu) by reacting an amino-protected 5-membered ring lactam (N-Boc-2-pyrrolidinone) with an amino acid ester (H-L-Ala-OtBu)

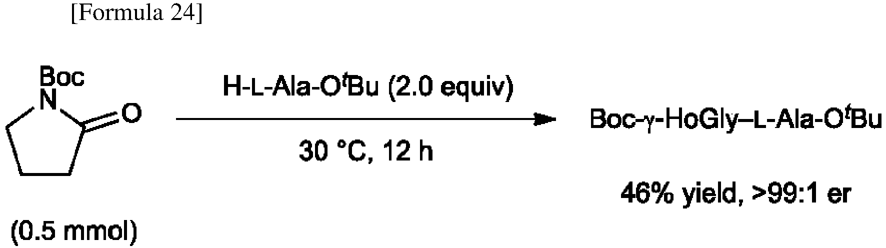

The same reaction procedure as Example III-1 was carried out except that 5-membered ring N-Boc-2-pyrrolidinone (N-Boc-γ-lactam) (92.61 mg, 0.5 mmol) was used as an amino-protected lactam, to thereby produce the title compound as colorless and transparent liquid (yield: 46%, >99:1 er).

### (3) Example III-3: Synthesis of a peptide (Boc-ε-HoGly-L-Ala-OtBu) by reacting an amino-protected 7-membered ring lactam (N-Boc-2-azacycloheptanone) with an amino acid ester (H-L-Ala-OtBu)

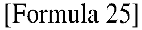

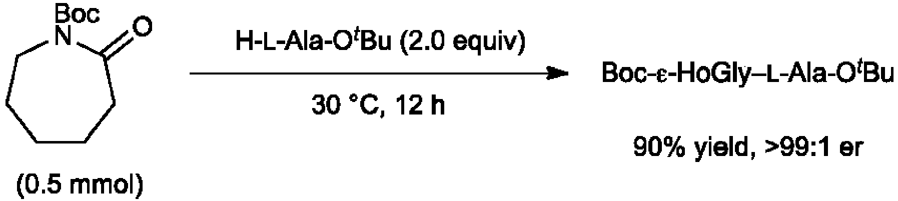

The same reaction procedure as Example III-1 was carried out except that 7-membered ring N-Boc-2-azacycloheptanone (N-Boc-ε-caprolactam) (106.64 mg, 0.5 mmol) was used as an amino-protected lactam, to thereby produce the title compound as colorless and transparent liquid (yield: 90%, >99:1 er).

### (4) Example III-4: Synthesis of a peptide (Boc-Gly-L-Ala-OtBu) by reacting an amino-protected lactam having two endocyclic peptide structures (N-Boc-2,5-piperazinedione) with an amino acid ester (H-L-Ala-OtBu)

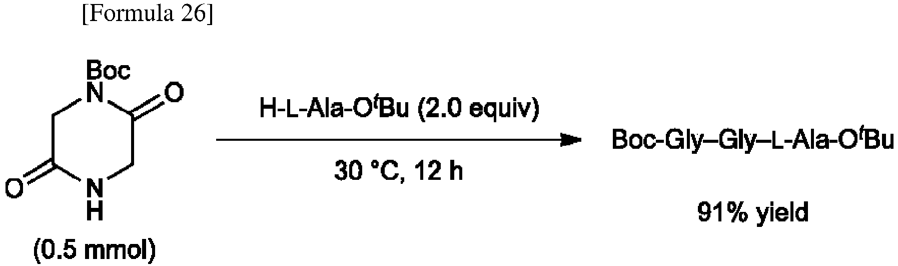

The same reaction procedure as Example III-1 was carried out except that N-Boc-2,5-piperazinedione (107.12 mg, 0.5 mmol) was used as an amino-protected lactam having two endocyclic peptide structures, to thereby produce the title compound as white solid (yield: 91%, >99:1 er).

### (5) Example III-5: Synthesis of a peptide (Boc-L-Glu(L-Ala-OtBu)-OtBu) by reacting an amino-protected lactam having a carboxylate ester group (N-Boc-pyroglutamate t-butyl ester) with an amino acid ester (H-L-Ala-OtBu)

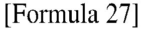

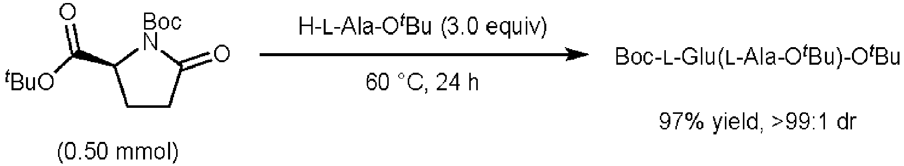

The same reaction procedure as Example III-1 was carried out except that N-Boc-pyroglutamic acid (142.67 mg, 0.5 mmol) was used as an amino-protected lactam having a carboxylate ester group, the amount of H-L-Ala-OtBu used was changed to 217.8mg (1.5 mmol), the reaction temperature was changed to 60 °C, and the reaction time was changed to 24 hours, to thereby produce the title compound as white solid (yield: 97%, >99:1 dr).

### (6) Example III-6: Synthesis of a peptide (Boc-L-Ala-L-Ala-L-Ala-OtBu) by reacting an amino-protected lactam having two endocyclic peptide structures and two methyl groups (N-Boc-cyclo(L-Ala-L-Ala)) with an amino acid ester (H-L-Ala-OtBu)

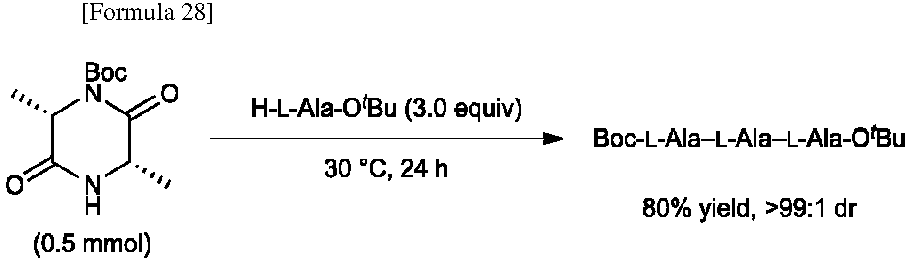

The same reaction procedure as Example III-1 was carried out except that N-Boc-cyclo(L-Ala-L-Ala) (121.14 mg, 0.5 mmol) was used as an amino-protected lactam having two endocyclic peptide structures and two methyl groups, the amount of H-L-Ala-OtBu used was changed to 217.8mg (1.5 mmol), and the reaction time was changed to 24 hours, to thereby produce the title compound as white solid (yield: 80%, >99:1 dr).

### (7) Example III-7: Synthesis of a peptide (Boc-Gly-L-Pro-L-Ala-OtBu) by reacting an amino-protected lactam having a condensed ring structure (Boc-cyclo(Gly-L-Pro)) with an amino acid ester (H-L-Ala-OtBu)

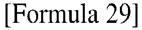

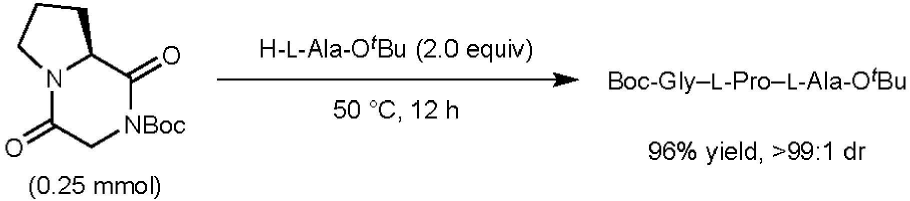

The same reaction procedure as Example III-1 was carried out except that Boc-cyclo(Gly-L-Pro) (63.57 mg, 0.25 mmol) was used as an amino-protected lactam having a condensed ring structure, and the amount of H-L-Ala-OtBu used was changed to 72.6mg (0.50 mmol), to thereby produce the title compound as white solid (yield: 96%, >99:1 r).

### (8) Example III-8: Synthesis of a peptide (Boc-βδ-HoGly-L-Thr(tBu)-OtBu) by reacting an amino-protected lactam (N-Boc-2-piperidone) with another amino acid ester (H-L-Thr(tBu)-OtBu)

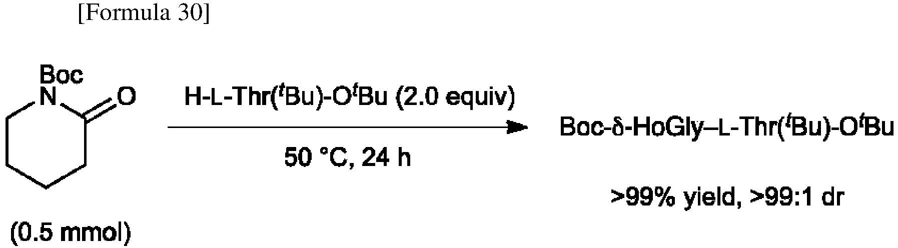

The same reaction procedure as Example III-1 was carried out except that N-Boc-2-piperidone (N-Boc-δ-lactam) (99.625 mg, 0.50 mmol) was used as an amino-protected lactam, H-L-Thr(tBu)-OtBu (231.3 mg, 1.00 mmol) was used as an amino acid ester, and the reaction was carried out at 50 °C for 24 hours, to thereby produce the title compound as white solid colorless and transparent liquid (yield: >99%, >99:1 dr).

### (9) Example III-9: Synthesis of a peptide (Boc-βδ-HoGly-L-Ile-OtBu) by reacting an amino-protected lactam (N-Boc-2-piperidone) with another amino acid ester (H-L-Ile-OtBu)

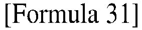

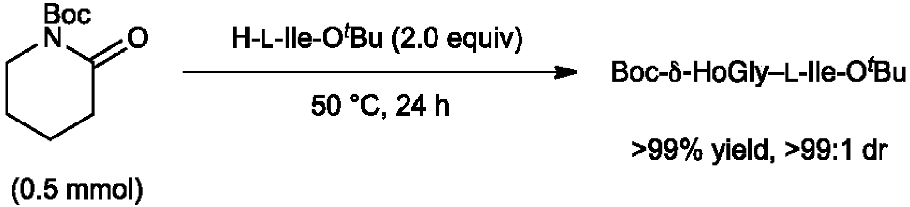

The same reaction procedure as Example III-1 was carried out except that N-Boc-2-piperidone (N-Boc-δ-lactam) (99.625 mg, 0.50 mmol) was used as an amino-protected lactam, H-L-Ile-OtBu (187.28 mg, 1.00 mmol) was used as an amino acid ester, and the reaction was carried out at 50 °C for 24 hours, to thereby produce the title compound as white solid to colorless and transparent liquid (yield: >99%, >99:1 dr).

### (10) Example III-10: Synthesis of a peptide (Boc-Gly-L-Pro-L-AlaSer(tBu)-OtBu) by reacting an amino-protected lactam having a condensed ring structure (Boc-cyclo(Gly-L-Pro)) with another amino acid ester (H-L-AlaSer(tBu)-OtBu)

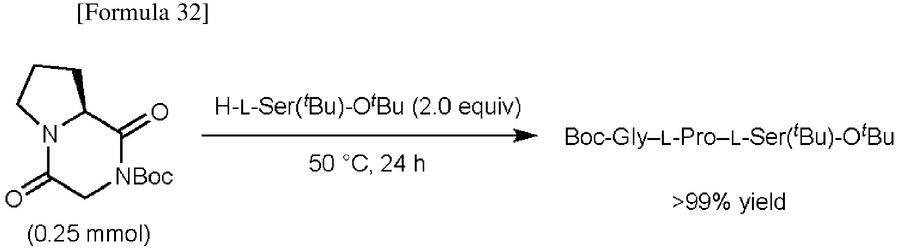

The same reaction procedure as Example III-1 was carried out except that Boc-cyclo(Gly-L-Pro) (63.57 mg, 0.25 mmol) was used as an amino-protected lactam having a condensed ring structure, H-L-Ser(tBu)-OtBu (108.66 mg, 0.50 mmol) was used as an amino acid ester, and the reaction was carried out at 50 °C for 24 hours, to thereby produce the title compound as white solid (yield: >99%, >99:1 dr).

### [Example Group IV: Peptide synthesis by reacting amino-protected lactams having various amino protective groups with amino acid esters]

### (1) Example IV-1: Synthesis of a peptide (Boc-Gly-L-Ala-O-tB) by reacting a lactam having two amino protective groups (N,N'-di-Boc-2,5-piperazinedione) with an amino acid ester (H-L-Ala-OtBu)

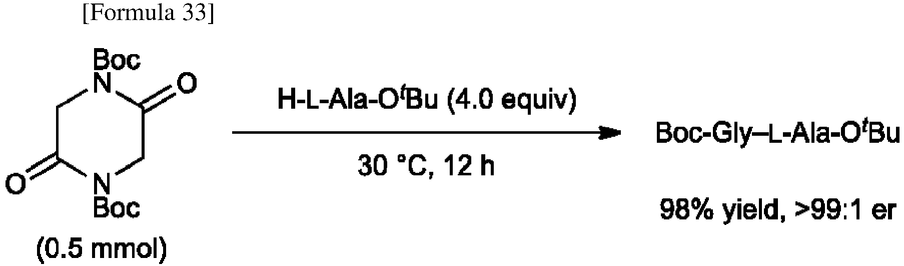

The same reaction procedure as Example I-1 was carried out except that N,N'-di-Boc-2,5-piperazinedione (157.17 mg, 0.5 mmol) was used as an amino-protected lactam having two Boc groups, and the amount of H-L-Ala-OtBu used was changed to 290.4mg (2.0 mmol), to produce Boc-Gly-L-Ala-OtBu as colorless and transparent liquid (yield: 98%, >99:1 er).

### (2) Example IV-2: Synthesis of a peptide (Boc-Gly-L-Val-OtBu) by reacting a lactam having two amino protective groups (N,N'-di-Boc-2,5-piperazinedione) with an amino acid ester (H-L-Val-OtBu)

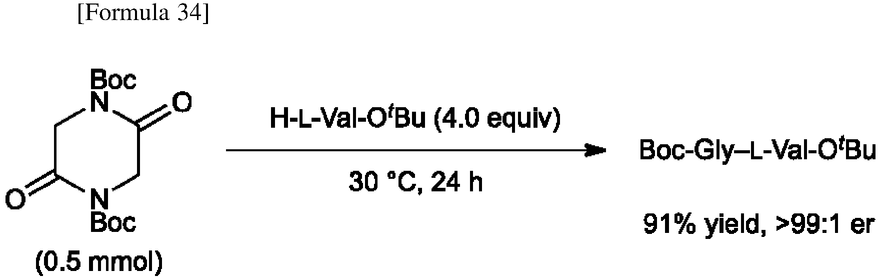

The same reaction procedure as Example IV-1 was carried out except that H-L-Val-OtBu (346.6 mg, 2.0 mmol) was used as an amino acid ester, and the reaction time was changed to 24 hours, to thereby produce the title compound as colorless and transparent liquid (yield: 91%, >99:1 er).

### (3) Example IV-3: Synthesis of a peptide (Cbz-δ-HoGly-L-Ala-OtBu) by reacting a lactam having Cbz as an amino protective group (N-Cbz-2-piperidone (N-Cbz-δ-lactam)) with an amino acid ester (H-L-Ala-OtBu)

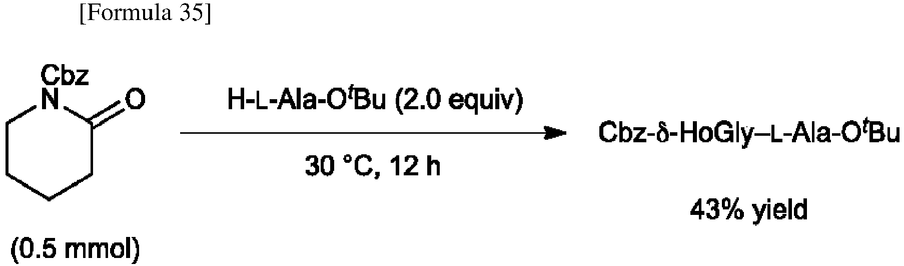

The same reaction procedure as Example IV-1 was carried out except that N-Cbz-2-piperidone (N-Cbz-δ-lactam) (116.63 mg, 0.50 mmol) was used as an amino-protected lactam having Cbz, and the reaction time was changed to 12 hours, to thereby produce the title compound as white solid (yield: 43%, >99:1 er).

### (4) Example IV-4: Synthesis of a peptide (Alloc-Gly-L-Thr(tBu)-OtBu) by reacting a lactam having two Alloc groups as amino protective groups (N,N'-di-Alloc-2,5-piperazinedione) with an amino acid ester (H-L-Thr(tBu)-OtBu)

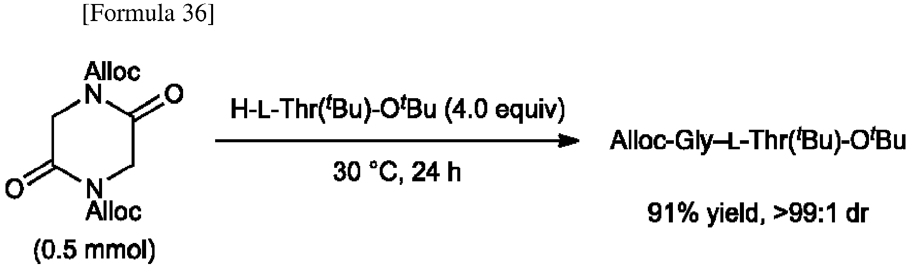

The same reaction procedure as Example IV-1 was carried out except that N,N'-di-Alloc-2,5-piperazinedione (141.1 mg, 0.50 mmol) was used as an amino-protected lactam having two Alloc groups, H-L-Thr(tBu)-OtBu (462.68 mg, 2.0 mmol) was used as an amino acid ester, and the reaction time was changed to 24 hours, to thereby produce the title compound as colorless and transparent liquid (yield: 91%, >99:1dr).

### [Example Group V: Other synthesis examples]

Peptides were synthesized by following each of the reaction procedures in Example Groups I to IV above, with making various changes in reaction conditions, such as the selection of the amino-protected lactam and/or the amino acid ester, reaction temperature, reaction time, the presence and absence and type of the solvent, and the presence and absence and selection of the catalyst. The reactions and results are summarized in the tables below.

**[Table 3-1]**

| No. | Reactions and results |
|---|---|
| 1 | 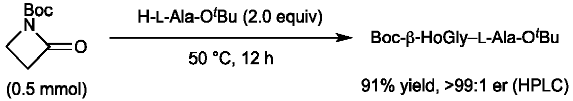 |
| 2 | 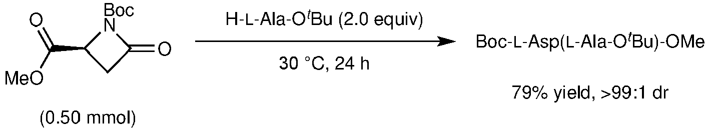 |
| 3 | 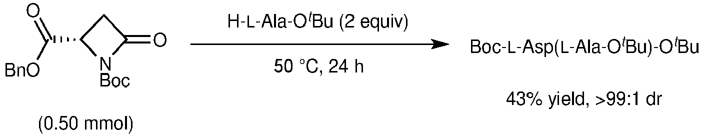 |
| 4 | 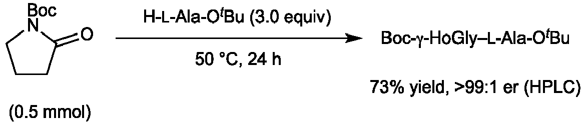 |
| 5 | 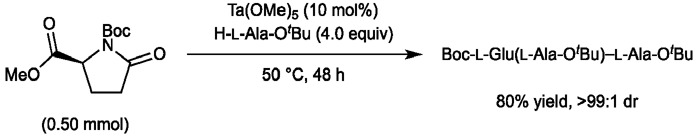 |
| 6 | 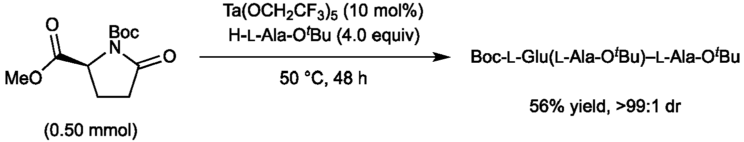 |
| 7 | 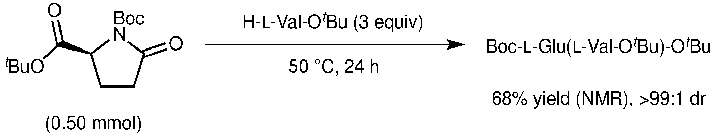 |
| 8 | 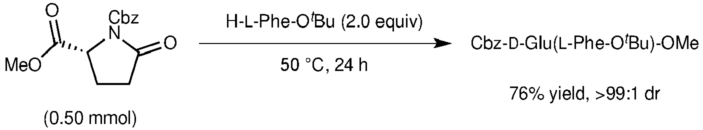 |

**[Table 3-2]**

| No. | Reactions and results |
|---|---|
| 9 | 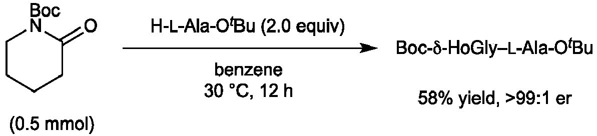 |
| 10 | 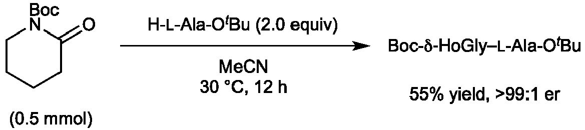 |
| 11 | 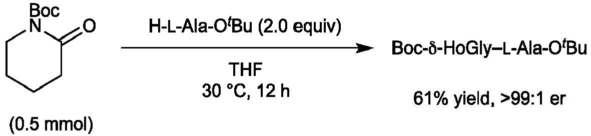 |
| 12 | 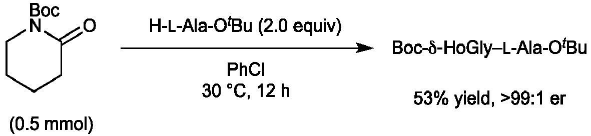 |
| 13 | 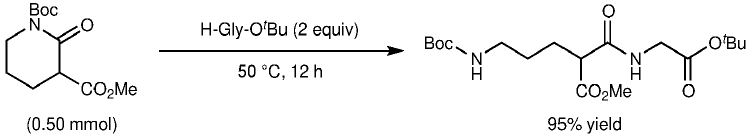 |
| 14 | 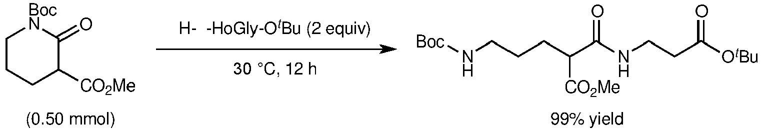 |
| 15 | 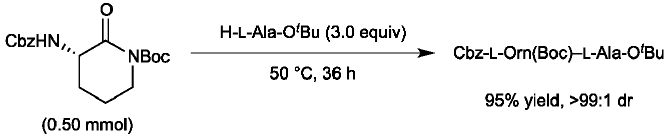 |
| 16 | 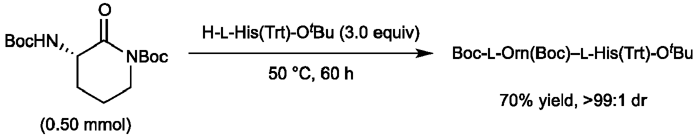 |
| 17 | 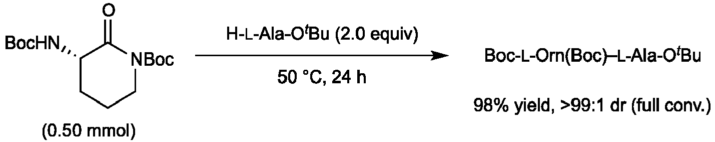 |

**[Table 3-3]**

| No. | Reactions and results |
|---|---|
| 18 | 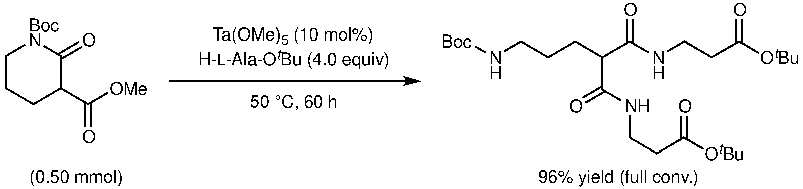 |
| 19 | 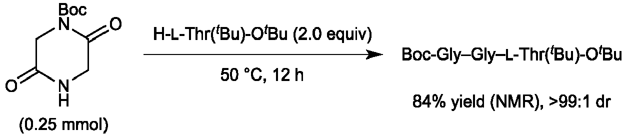 |
| 20 | 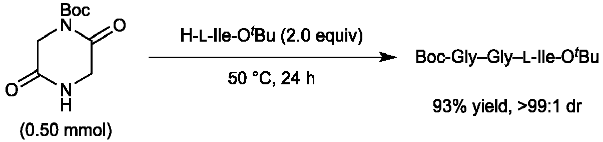 |
| 21 | 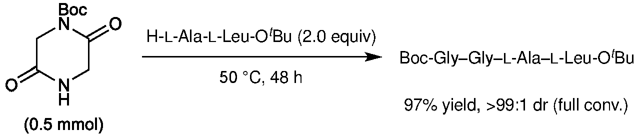 |
| 22 | 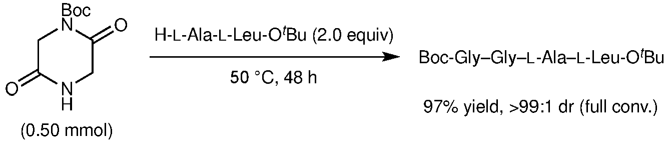 |
| 23 | 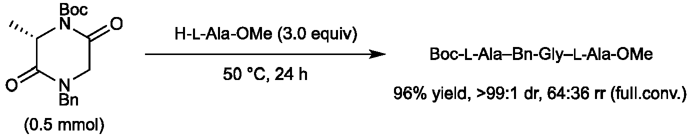 |
| 24 | 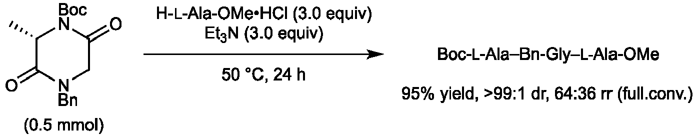 |

**[Table 3-4]**

| No. | Reactions and results |
|---|---|
| 25 | 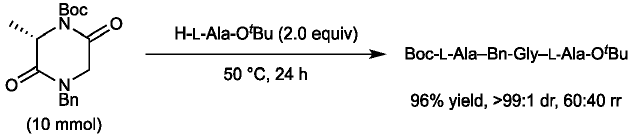 |
| 26 | 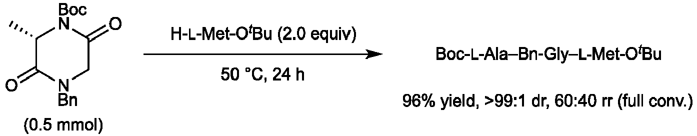 |
| 27 | 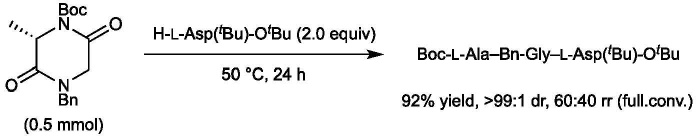 |
| 28 | 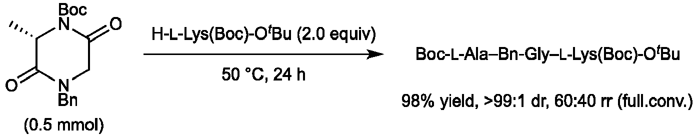 |
| 29 | 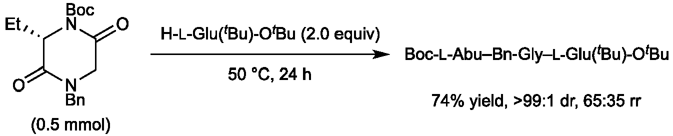 |
| 30 | 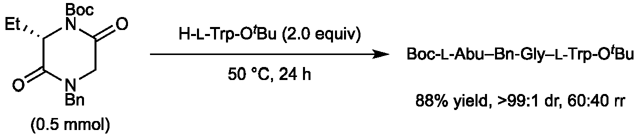 |

**[Table 3-5]**

| No. | Reactions and results |
|---|---|
| 31 | 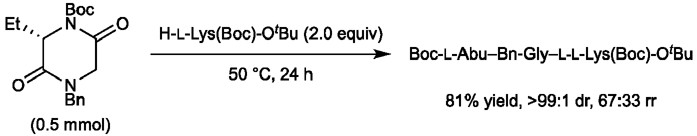 |
| 32 | 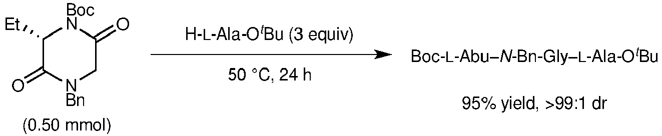 |
| 33 | 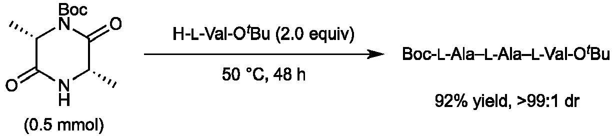 |
| 34 | 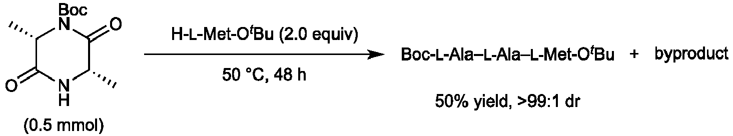 |
| 35 | 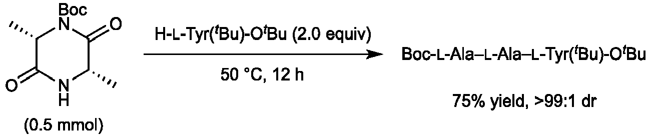 |
| 36 | 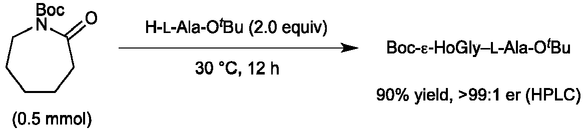 |
| 37 | 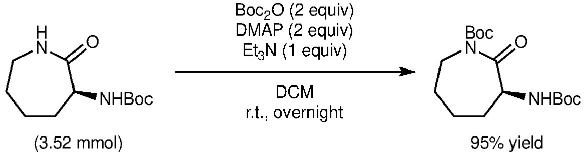 |
| 38 | 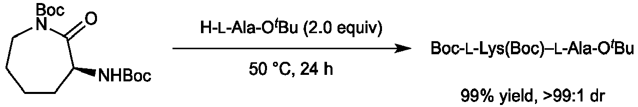 |

**[Table 3-6]**

| No. | Reactions and results |
|---|---|
| 39 | 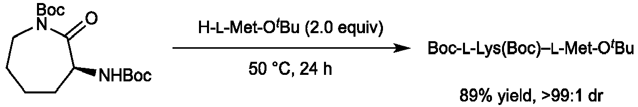 |
| 40 | 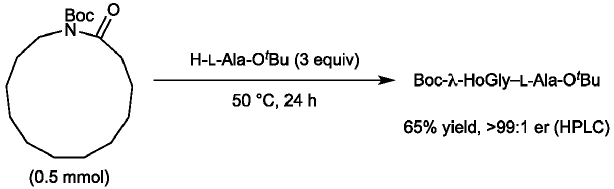 |
| 41 | 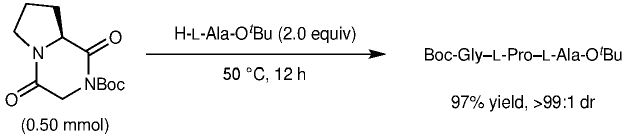 |
| 42 | 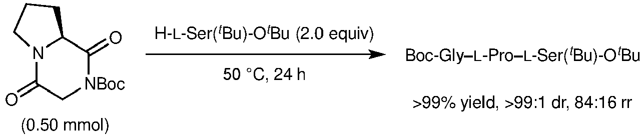 |
| 43 | 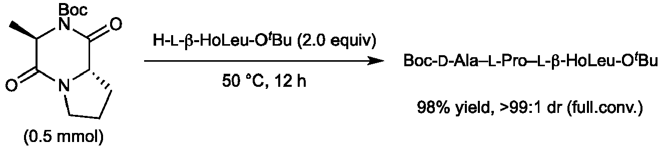 |

**[Table 3-7]**

| No. | Reactions and results |
|---|---|
| 44 | 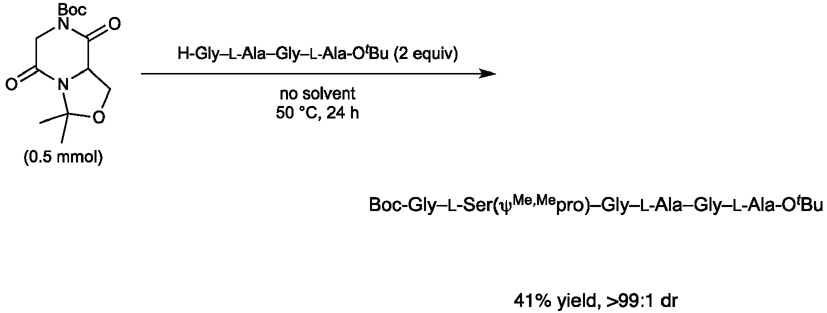 |
| 45 | 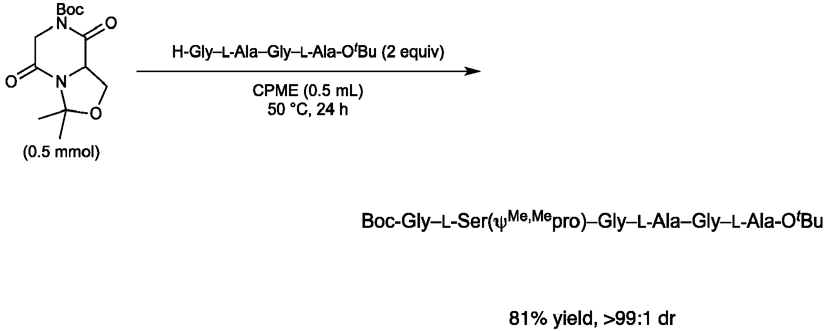 |
| 46 | 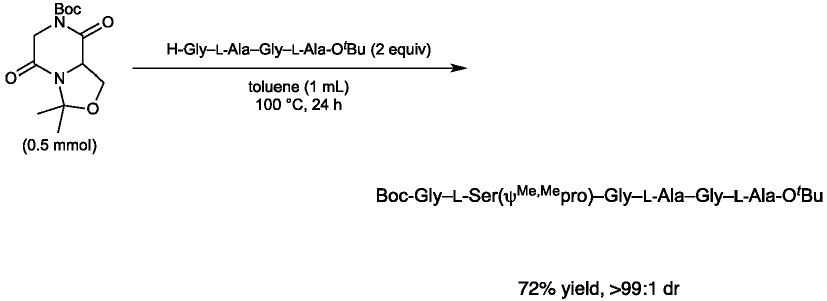 |

**[Table 3-8]**

| No. | Reactions and results |
|---|---|
| 47 | 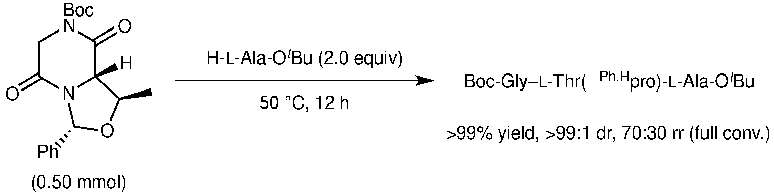 |
| 48 | 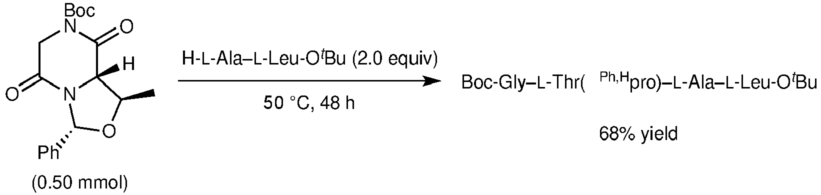 |
| 49 | 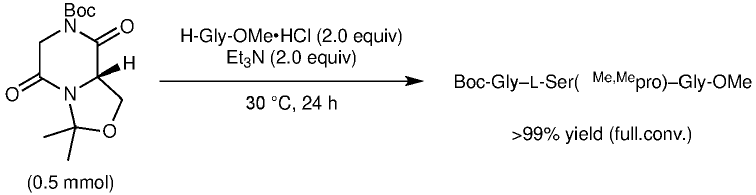 |
| 50 | 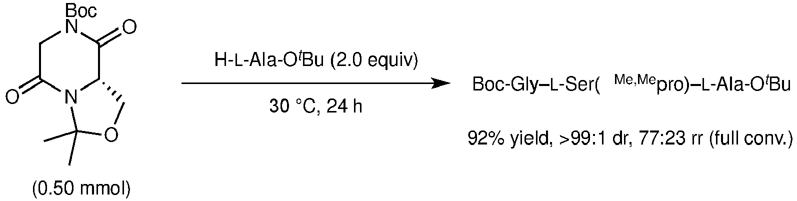 |

**[Table 3-9]**

| No. | Reactions and results |
|---|---|
| 51 | 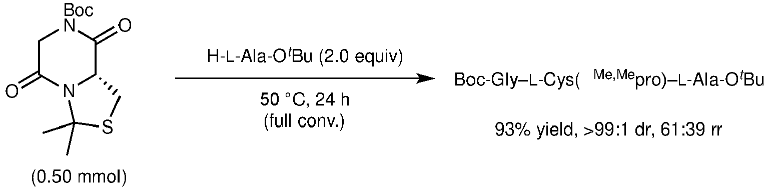 |
| 52 | 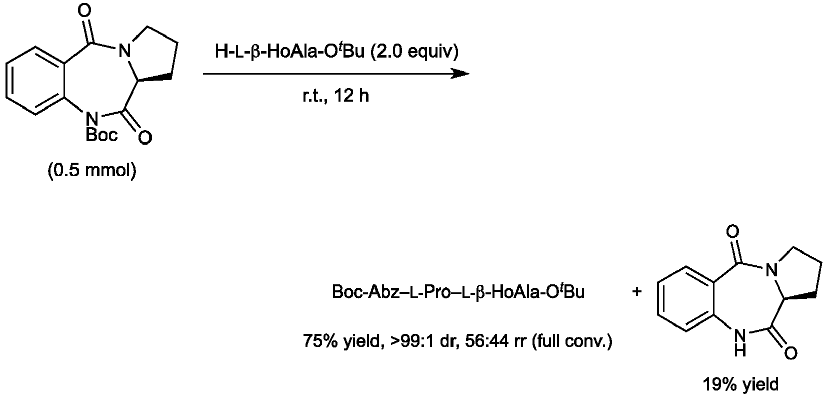 |
| 53 | 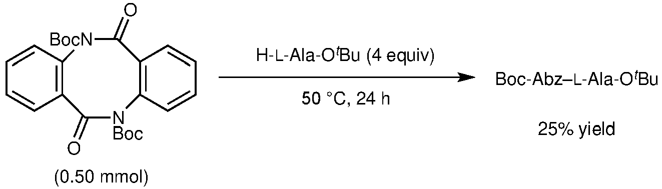 |

## Claims

1. A method for producing a polypeptide compound represented by formula (P), comprising causing amide forming reaction between an amino-protected lactam compound represented by formula (R1) and an amino acid ester or peptide ester compound represented by formula (R2). 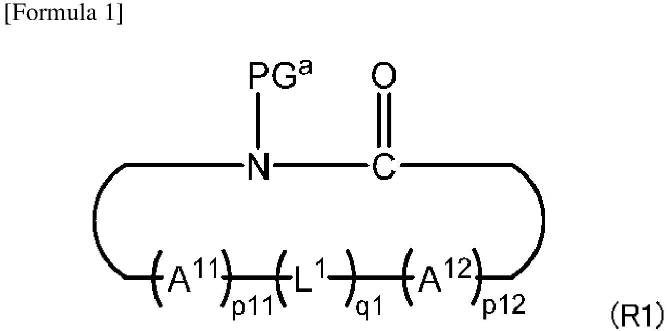 In formula (R1),
PG^{a} represents -C(=O)-O-R^{a}, -C(=O)-R^{a}, or -S(=O)₂-R^{a} a protective group represented by (where R^{a} represents a monovalent substituent.),
A¹¹and A¹² represents, independently of each other, a bivalent aliphatic hydrocarbon group having 1 to 5 carbon atoms which may optionally have one or more substituents,
p11 and p12 represents, independently of each other, 0 or 1,
L¹ represents a bivalent linking group,
q1 represents 0 or 1,
provided that the ring containing (A¹¹), (L¹), and (A¹²) is a 4- to 17-membered ring,
wherein the bivalent linking group L¹ may be combined with A¹¹ and/or A¹² to form a 4- to 8-membered heterocyclic ring that fuses with the lactam ring of formula (R1). 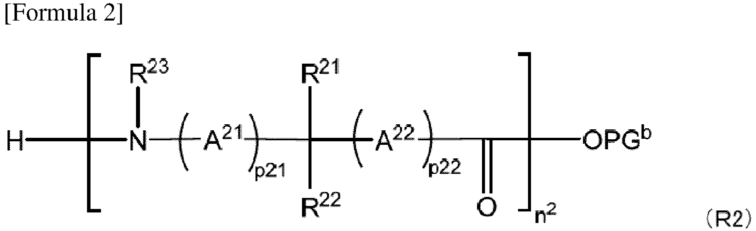
In formula (R2),
PG^{b} represents a protective group for carboxyl groups,
R²¹and R²² represents, independently of each other, a hydrogen atom, halogen atom, hydroxyl group, carboxyl group, nitro group, cyano group, or thiol group, and which may optionally have one or more substituents, amino group, monovalent aliphatic hydrocarbon group, monovalent aromatic hydrocarbon group, or monovalent heterocyclic group,
R²³ represents a hydrogen atom, carboxyl group, hydroxyl group, and which may optionally have one or more substituents monovalent aliphatic hydrocarbon group, aromatic hydrocarbon group, or heterocyclic group, provided that when R²³ is a monovalent aliphatic hydrocarbon group, aromatic hydrocarbon group, or heterocyclic group, R²³ may be bound to the nitrogen atom via a linking group, or
alternatively, R²¹ and R²³ may bind to each other and, along with the carbon atom to which R²¹ binds and the nitrogen atom to which R²³ binds, form a heterocyclic ring which may optionally have one or more substituents,
A²¹ and A²² represents, independently of each other, a bivalent aliphatic hydrocarbon group having 1 to 3 carbon atoms which may optionally have one or more substituents,
p21 and p22 represents, independently of each other, 0 or 1, and
n² represents an integer of 1 or higher corresponding to the number of the structure units parenthesized with [ ], provided that when is n² equal to or greater than 2, then the two or more structure units in [ ] may be either identical to each other or different from each other. 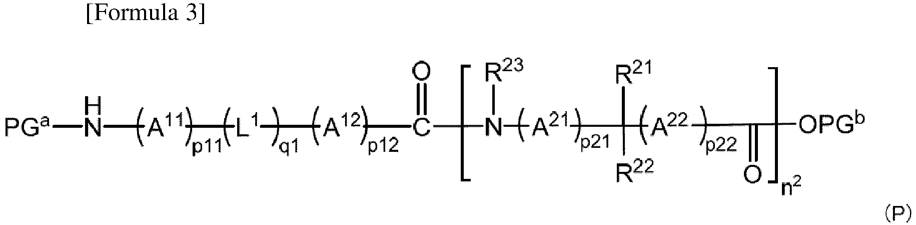
In formula (P),
PG^{b}, A¹¹, A¹², L¹, p11, p12, and q1 each represent the same definition as that of the same symbol in formula (R1), and
PG^{b}, R²¹, R²², R²³, A²¹, A²², p21, p22, and n² each represent the same definition as that of the same symbol in the formula (R2).

2. The method according to claim 1, wherein in formula (R1), PG^{a} is a protective group selected from a tert-butoxy carbonyl group (Boc), benzyloxycarbonyl group (Cbz), 2,2,2-trichloroethoxy carbonyl (Troc) group, allyoxycarbonyl (Alloc) group, 9-fluorenyl methyloxycarbonyl (Fmoc) group, 2-(trimethylsilyl)ethoxy carbonyl (Teoc) group, benzoyl (Bz) group, phthaloyl (Phth) group, paramethoxybenzoyl group (PMPCO), cinnamoyl group, toluene sulfonyl (Ts) group, ands 2- or 4-nitrobenzene sulfonyl (Ns) group.

3. The method according to claim 1 or 2, wherein in formula (R1), p11 is 1, p12 and q1 are both 0, A¹¹ represents a bivalent aliphatic hydrocarbon group having 2 to 6 carbon atoms which may optionally have one or more substituents.

4. The method according to claim 1 or 2, wherein in formula (R1), p11, p12, and q1 are all 1, A¹¹and A¹² are, independently of each other, a bivalent aliphatic hydrocarbon group having 1 to 3 carbon atoms which may optionally have one or more substituents, and L¹ is -C(=O)-N-.

5. The method according to any one of claims 1 to 4, wherein the molar ratio of the lactam compound of formula (R1) to the amino acid ester or peptide ester compound of formula (R2) used is from 1:5 to 5:1.

6. The method according to any one of claims 1 to 5, wherein the reaction is a batch reaction or a flow reaction.
